# EUROPÄISCHE PATENTSCHRIFT

(11) **EP 4 359 372 B1**
(45) Veröffentlichungstag und Bekanntmachung des Hinweises auf die Patenterteilung: **25.06.2025**
(21) Anmeldenummer: 22733377.0
(22) Anmeldetag: 10.06.2022
(51) Int. Cl.: C07B 31/00, C07D 307/44, C07F 9/50, C07F 13/00, C07D 307/92, C07C 29/149

(54) **HYDRIERUNG VON ESTERN ZU ALKOHOLEN IN GEGENWART EINES MN-PNN-KOMPLEXES**
HYDROGENATION OF ESTERS TO ALCOHOLS IN THE PRESENCE OF AN MN-PNN COMPLEX
HYDROGÉNATION D'ESTERS EN ALCOOLS EN PRÉSENCE D'UN COMPLEXE DE MN-PNN

(30) Priorität: 21.06.2021 EP 21180529
(43) Veröffentlichungstag der Anmeldung: 01.05.2024
(73) Patentinhaber: BASF SE, 67056 Ludwigshafen am Rhein (DE)
(72) Erfinder: SCHAUB, Thomas, 67056 Ludwigshafen (DE); SCHELWIES, Mathias, 67056 Ludwigshafen (DE); ZUBAR, Viktoriia, 69120 Heidelberg (DE)
(74) Vertreter: BASF IP Association
(86) Internationale Anmeldenummer: PCT/EP2022/065787
(87) Internationale Veröffentlichungsnummer: WO 2022/268525

(56) Entgegenhaltungen:
- WO-A1-2019/138216
- WO-A1-2021/001240
- DAS UTTAM KUMAR ET AL: "Manganese catalyzed selective hydrogenation of cyclic imides to diols and amines", GREEN CHEMISTRY, vol. 22, no. 10, 26 May 2020 (2020-05-26), GB, pages 3079 - 3082, XP055972042, ISSN: 1463-9262, DOI: 10.1039/D0GC00570C
- TRAUT-JOHNSTONE TELISHA ET AL: "Heteroditopic P,N ligands in gold(I) complexes: Synthesis, structure and cytotoxicity", JOURNAL OF INORGANIC BIOCHEMISTRY, ELSEVIER INC, US, vol. 145, 30 January 2015 (2015-01-30), pages 108 - 120, XP029222294, ISSN: 0162-0134, DOI: 10.1016/J.JINORGBIO.2015.01.014
- ZUBAR VIKTORIIA ET AL: "Manganese-Catalyzed Hydrogenation of Sclareolide to Ambradiol", CHEMCATCHEM, vol. 14, no. 1, 10 January 2022 (2022-01-10), XP055971976, ISSN: 1867-3880, Retrieved from the Internet <URL:https://onlinelibrary.wiley.com/doi/full-xml/10.1002/cctc.202101443> DOI: 10.1002/cctc.202101443
- LI XIAO-GEN ET AL: "Hydrogenation of Esters by Manganese Catalysts", ADVANCED SYNTHESIS AND CATALYSIS, vol. 364, no. 4, 15 February 2022 (2022-02-15), pages 744 - 749, XP055972011, ISSN: 1615-4150, Retrieved from the Internet <URL:https://onlinelibrary.wiley.com/doi/full-xml/10.1002/adsc.202101376> DOI: 10.1002/adsc.202101376

## Beschreibung

Die vorliegende Erfindung betrifft ein Verfahren zur Hydrierung eines Esters mit molekularem Wasserstoff zu den korrespondierenden Alkoholen in Gegenwart eines Mangan-Komplexes mit einem dreizähnigen PNN-Liganden.

Alkohole sind nicht nur wichtige Lösungsmittel, sondern auch wichtige Zwischenprodukte und Synthesebausteine, etwa zur Herstellung von Arznei- und Pflanzenschutzmitteln oder Riechstoffen. In Abhängigkeit von der Art des gewünschten Alkohols und der Verfügbarkeit des entsprechenden Ausgangsstoffes sind oftmals die direkte Hydrierung des entsprechenden Esters mit Wasserstoff oder auch die Reduktion mit Reduktionsmitteln die Methoden der Wahl.

Die Synthese von Alkoholen aus Estern erfolgt üblicherweise durch den Einsatz von Metallhydriden wie beispielsweise LiAlH₄ oder NaBH₄, durch heterogenkatalytische Hydrierung mit Wasserstoff, oder durch homogenkatalytische Hydrierung mit Wasserstoff. Homogenkatalytische Hydrierungen mit Wasserstoff ermöglichen oft weniger drastischen Reaktionsbedingungen bei gleichzeitig besserer Selektivität. Insbesondere der Einsatz von Ruthenium-Komplexen mit mehrzähnigen Phosphor-, Schwefel- und Stickstoff-haltigen Liganden hat sich gemäß dem Stand der Technik hierzu bewährt, in den letzten Jahren wurden aber auch Alternativen entwickelt, die Mangan als aktives Metall enthalten.

So beschreiben Beller et al. in Angewandte Chemie International Edition 2016, Vol. 55, Seiten 15364-15368 den Einsatz von Mn-Komplexen mit einem sogenannten Pincer-Liganden vom Typ PNP zur Hydrierung von Estern zu Alkoholen. Eingesetzt werden hierbei Liganden, die im Zentrum eine NH-Einheit aufweisen, an welcher sich zwei Ethyl-Dialkyl-phosphineinheiten befinden. Das Mangan-Atom liegt in der Oxidationsstufe +I vor und trägt noch mindestens zwei Carbonylliganden. Mit diesen Katalysatoren lassen sich eine Reihe von Estern und Lactonen zu den entsprechenden Alkoholen bzw. Diolen hydrieren.

Weitere Mn-Katalysatoren zur Hydrierung von Estern zu Alkoholen sind bekannt aus WO2019/138216 und aus der Publikation von Das Uttam Kumar et al in Green Chemistry 2020, 22(10), 3079-3082.

Nachteilig an diesen Katalysatoren sind die relativ hohen Katalysatorladungen von 2 mol% sowie die 10 mol% an KOtBu als notwendigen Cokatalysator, die eingesetzt werden müssen, um hohe Umsätze in der Hydrierung zu erhalten.

Milstein et al. beschreiben in Chemistry, a European Journal 2017, Vol. 23, Seiten 5934-5938 den Einsatz von Mn-Komplexen mit einem sogenannten Pincer-Liganden vom Typ PNN zur Hydrierung von Estern zu Alkoholen. Der beschriebene tridentate Pincer-Ligand weist eine Pyridyl-Gruppe als Rückgrat und als Donor-Gruppen eine Phosphino- und NHR-Gruppe mit einer Alkylgruppe auf. Mit diesen Katalysatoren lassen sich eine Reihe von Estern und Lactonen zu den entsprechenden Alkoholen bzw. Diolen hydrieren.

Nachteilig am Einsatz der genannten Pincer-Liganden des Typs PNN ist deren aufwändige, mehrstufige Synthese unter Verwendung anspruchsvoller Reagenzien wie nButyllithium ausgehend von 2,6-Dimethylpyridin. Nachteilig an diesen Katalysatoren sind ebenfalls die relativ hohen Katalysatorladungen von mindestens 1mol% sowie die Verwendung des teuren und schwierig zu handhabenden KH als notwendigen Cokatalysator, die eingesetzt werden müssen, um hohe Umsätze in der Hydrierung zu erhalten. Mit den einfacheren und günstigeren Alkoholatbasen lassen sich mit diesem Mangankatalysator nur geringe Umsätze erzielen.

Pidko et al. beschreiben in Angewandte Chemie International Edition 2017, Vol. 56, Seiten 7531-7534 den Einsatz von Mn-Komplexen mit einfachen bidentate PN-Liganden zur Hydrierung von Estern zu Alkoholen. Eingesetzt werden hierbei Liganden, eine NH₂-Einejit, eine Ethylenbrücke und eine PR₂ (R = Alkyl, Aryl) Gruppe aufweisen. Das Mangan-Atom liegt in der Oxidationsstufe +I vor und trägt noch mindestens zwei Carbonylliganden. Vorteilhaft ist hier, dass die Liganden einfach herzustellen sind.

Allerdings ist mit diesen Katalysatoren nur die Hydrierung von acyclische Estern und nicht Lactone beschrieben. Weitere Nachteile dieser Katalysatoren sind die relativ hohen Katalysatorladungen von 1 mol% sowie nötigen sehr hohen Beladungen von 10-75 mol% an KOtBu als notwendigen Kokatalysator, die eingesetzt werden müssen, um hohe Umsätze in der Hydrierung zu erhalten. Umsätze des Esters über 90% lassen sich hier nur mit Basenladungen von mindestens 50 mol% erzielen, was nachteilig für ein wirtschaftliches Verfahren ist.

Clark et al. beschreiben in Organic Letters 2018, Vol. 20, Seiten 2654-2658 den Einsatz von Mn-Komplexen mit einem sogenannten Pincer-Liganden vom Typ PNN zur Hydrierung von Estern zu Alkoholen. Der beschriebene tridentate Pincer-Ligand weist eine NH-Gruppe als Rückgrat und als Donor-Gruppen eine Pyridyl- und sowie eine über Ferrocen verbrückte PPh₂-Gruppe auf. Das Mangan-Atom liegt in der Oxidationsstufe +I vor und trägt noch drei Carbonylliganden. Vorteilhaft ist hier, dass die Liganden einfach herzustellen sind. Mit diesem Katalysator lassen sich eine Reihe von Estern und Lactonen zu den entsprechenden Alkoholen bzw. Diolen hydrieren.

Vorteilhaft an diesem Katalysator die geringer Katalysatorladungen von nur 0.1mol% die nötig sind, um zum Beispiel das Lacton Sclareolid zu moderaten Ausbeuten von 75% des entsprechenden Diols zu hydrieren.

Nachteilig am Einsatz diese Pincer-Liganden des Typen PNN ist seine sehr aufwändige, mehrstufige Synthese unter Verwendung anspruchsvoller Reagenzien wie nButyllithium.

Nachteilig an diesem Katalysator ist auch noch die relativ hohe notwendige Beladung von 10 mol% Base als Cokatalysatoren, wie beim Lacton Sclareolid, um zu guten Umsätzen zu gelangen.

WO 2021/001240 A1 beschreibt den Einsatz von einfach herzustellenden PNN-Liganden, mit welchen sich hochaktive Rutheniumkatalysatoren zur Hydrierung von Estern herstellen lassen, bei denen man nur sehr wenig Base als Cokatalysator benötigt bzw. dieser auch ohne Basenzusatz aktiv sein kann. Über deren Eignung als Liganden für aktive Mangankatalysatoren war allerdings bislang nichts bekannt und strukturell weicht dieser von den oben beschriebenen Liganden für Mangankatalysatoren in der Esterhydrierung ab, weshalb nicht zu erwarten war, dass sich mit diesem Liganden hochaktive Mn-Katalysatoren erzeugen lassen.

Aufgabe der vorliegenden Erfindung war es, ein Verfahren zur homogenkatalysierten Hydrierung von Estern zu den korrespondierenden Alkoholen zu finden, welches die genannten Nachteile des Standes der Technik nicht oder in nur untergeordnetem Maße aufweist, hinsichtlich der erforderlichen Apparate und Reaktionsbedingungen einfach durchzuführen ist und eine möglichst hohe Raum-Zeit-Ausbeute ermöglicht.

Insbesondere soll der katalytisch aktive Komplex direkt aus gut verfügbaren Einsatzstoffen herstellbar sein, eine hohe Aktivität in der Hydrierung von Estern zu Alkoholen aufweisen und möglichst wenig Cokatalysator zu benötigen und letztendlich auch ohne übermäßig hohen Aufwand zu entsorgen sein. In diesem Zusammenhang kommt vor allem dem komplexbildenden Liganden eine besondere Bedeutung zu, der möglichst einfach herzustellen ist, aber trotzdem einen Mangankatalysator mit hoher Aktivität erzeugt.

Überraschend wurde ein Verfahren zur Hydrierung eines Esters der allgemeinen Formel (III) 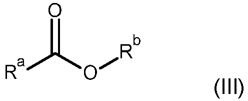
gefunden, bei dem die Reste R^{a} und R^{b} jeweils unabhängig voneinander für einen Kohlenstoff enthaltenden organischen, linearen oder verzweigten, nichtcyclischen oder cyclischen, gesättigten oder ungesättigten, aliphatischen, aromatischen oder aryliphatischen, unsubstituierten oder durch Heteroatome oder funktionelle Gruppen unterbrochenen oder substituierten Rest mit einer Molmasse von 15 bis 10000 g/mol stehen, wobei beide Reste R^{a} und R^{b} auch miteinander verbunden sein können,
mit molekularem Wasserstoff zu den Alkoholen 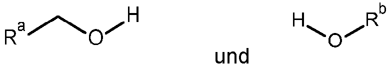
bei einer Temperatur von 50 bis 200°C und einem Druck von 0,1 bis 20 MPa abs in Gegenwart eines Mangan(I)-Komplexes (I), gefunden, bei dem der Mangan-Komplex einen dreizähnigen Liganden L mit der allgemeinen Formel (II) 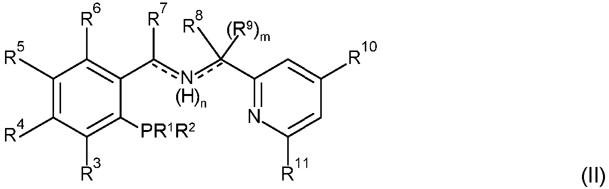
sowie mindestens zwei Carbonylliganden enthält, wobei
R¹, R² unabhängig voneinander für einen aliphatischen Kohlenwasserstoffrest mit 1 bis 8 Kohlenstoffatomen, einen aromatischen Kohlenwasserstoffrest mit 6 oder 10 Kohlenstoffatomen oder einen araliphatischen Kohlenwasserstoffrest mit 7 bis 12 Kohlenstoffatomen stehen, wobei die genannten Kohlenwasserstoffreste unsubstituiert oder mit 1 bis 3 Methoxy-, Thiomethoxy- oder Dimethylamino-Gruppen substituiert sind, und die beiden Reste R¹ und R² unter Bildung eines, das Phosphoratom einschließenden 5 bis 10-Ringes auch miteinander verbunden sein können,
R³, R⁴, R⁵, R⁶, R¹⁰, R¹¹ unabhängig voneinander für Wasserstoff, lineares C₁ bis C₄-Alkyl, verzweigtes C₃ bis C₄-Alkyl, Methoxy, Hydroxy, Trifluormethyl, Nitril oder Dialkylamino mit unabhängig voneinander 1 bis 4 Kohlenstoffatomen je Alkylgruppe, stehen,
R⁷, R⁸, R⁹ unabhängig voneinander für Wasserstoff, lineares C₁ bis C₄-Alkyl oder verzweigtes C₃ bis C₄-Alkyl stehen,
n, m unabhängig voneinander 0 oder 1 sind, und
die durchgezogen-gestrichelten Doppellinien für eine Einfach- oder Doppelbindung stehen, mit der Maßgabe, dass

| | |
|---|---|
| im Falle von n = 1 | beide durchgezogen-gestrichelten Doppellinien eine Einfachbindung darstellen und m gleich 1 ist, und |
| im Falle von n = 0 | eine durchgezogen-gestrichelte Doppellinie eine Einfachbindung und die andere durchgezogen-gestrichelte Doppellinie eine Doppelbindung dar stellt, wobei im Falle einer Doppelbindung auf der dem Phenylring zugewandten Seite m = 1 ist, im Falle einer Doppelbindung auf der dem Pyridylring zugewandten Seite m = 0 ist, oder beide durchgezogen-gestrichelten Doppellinien eine Einfachbindung darstellen und m gleich 1 ist. |

Kern des erfindungsgemäßen Verfahrens ist der Einsatz eines Mangan(I)-Komplexes , welcher einen dreizähnigen Liganden L der allgemeinen Formel (II) sowie mindestens zwei Carbonylliganden enthält, in der Hydrierung von Estern mit molekularem Wasserstoff zu den korrespondierenden Alkoholen.

Bei dem dreizähnigen Liganden L handelt es sich um einen sogenannten PNN-Liganden mit der allgemeinen Formel (II) 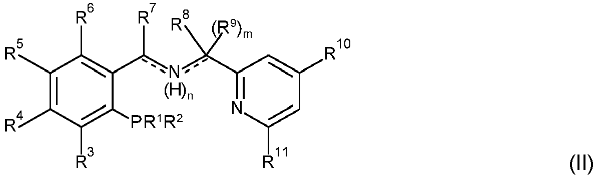 in der
R¹, R² unabhängig voneinander für einen aliphatischen Kohlenwasserstoffrest mit 1 bis 8 Kohlenstoffatomen, einen aromatischen Kohlenwasserstoffrest mit 6 oder 10 Kohlenstoffatomen oder einen araliphatischen Kohlenwasserstoffrest mit 7 bis 12 Kohlenstoffatomen stehen, wobei die genannten Kohlenwasserstoffreste unsubstituiert oder mit 1 bis 3 Methoxy-, Thiomethoxy- oder Dimethylamino-Gruppen substituiert sind, und die beiden Reste R¹ und R² unter Bildung eines, das Phosphoratom einschließenden 5 bis 10-Ringes auch miteinander verbunden sein können,
R³, R⁴, R⁵, R⁶, R¹⁰, R¹¹ unabhängig voneinander für Wasserstoff, lineares C₁ bis C₄-Alkyl, verzweigtes C₃ bis C₄-Alkyl, Methoxy, Hydroxy, Trifluormethyl, Nitril oder Dialkylamino mit unabhängig voneinander 1 bis 4 Kohlenstoffatomen je Alkylgruppe, stehen,
R⁷, R⁸, R⁹ unabhängig voneinander für Wasserstoff, lineares C₁ bis C₄-Alkyl oder verzweigtes C₃ bis C₄-Alkyl stehen,
n, m unabhängig voneinander 0 oder 1 sind, und
die durchgezogen-gestrichelten Doppellinien für eine Einfach- oder Doppelbindung stehen, mit der Maßgabe, dass

| | |
|---|---|
| im Falle von n = 1 | beide durchgezogen-gestrichelten Doppellinien eine Einfachbindung darstellen und m gleich 1 ist, und |
| im Falle von n = 0 | eine durchgezogen-gestrichelte Doppellinie eine Einfachbindung und die andere durchgezogen-gestrichelte Doppellinie eine Doppelbindung dar stellt, wobei im Falle einer Doppelbindung auf der dem Phenylring zugewandten Seite m = 1 ist, im Falle einer Doppelbindung auf der dem Pyridylring zugewandten Seite m = 0 ist, oder beide durchgezogen-gestrichelten Doppellinien eine Einfachbindung darstellen und m = 1 ist. |

Dreizähnig bedeutet, dass Ligand L (II) drei Koordinationsstellen im Mangan(I)-Komplex (I) besetzt. Bei den drei Liganden-Donoratomen handelt es sich um das P und die beiden N-Atome, woraus sich auch die Bezeichnung PNN-Ligand ableitet.

Hinsichtlich der Umgebung des mittleren Donoratoms kann der Ligand grundsätzlich vier verschiedene Substrukturen aufweisen, die im Folgenden näher erläutert sind.
(1) Im Falle von n = 1 stellen beide durchgezogen-gestrichelte Doppellinien eine Einfachbindung dar und m beträgt 1. Daraus resultiert die allgemeine Formel (IIa). Ligand (IIa) ist neutral, besitzt also die Ladung "0". 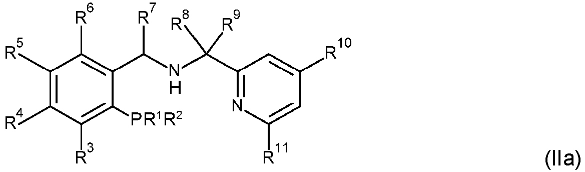 Im Falle von n = 0 gibt es insgesamt drei verschiedene Substrukturen.
(2) Ist n = 0 und die dem Phenylring zugewandte durchgezogen-gestrichelte Doppellinie eine Doppelbindung und die dem Pyridylring zugewandte durchgezogen-gestrichelte Doppellinie eine Einfachbindung, so ist m gleich 1. Daraus resultiert die allgemeine Formel (IIb). Ligand (IIb) ist neutral, besitzt also die Ladung "0". 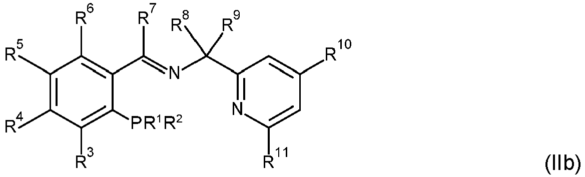
(3) Ist n = 0 und die dem Pyridylring zugewandte durchgezogen-gestrichelte Doppellinie eine Doppelbindung und die dem zugewandte Phenylring durchgezogen-gestrichelte Doppellinie eine Einfachbindung, so ist m gleich 0. Daraus resultiert die allgemeine Formel (IIc). Ligand (IIc) ist neutral, besitzt also die Ladung "0". 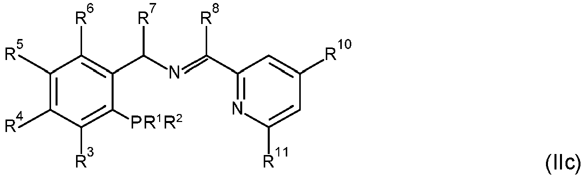
(4) Bei der vierten Variante ist ebenfalls n = 0, jedoch sind beide durchgezogen-gestrichelten Doppellinien Einfachbindungen und m ist 1. Das N-Atom weißt somit eine negative Ladung auf. Daraus resultiert die allgemeine Formel (IId). Der Ligand (IId) besitzt somit die Ladung "-1".

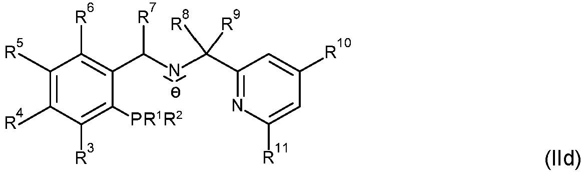

Die Reste R¹ und R² des Liganden (II) können in einem weiten Umfang variieren und stehen unabhängig voneinander für einen aliphatischen Kohlenwasserstoffrest mit 1 bis 8 Kohlenstoffatomen, einen aromatischen Kohlenwasserstoffrest mit 6 oder 10 Kohlenstoffatomen oder einen araliphatischen Kohlenwasserstoffrest mit 7 bis 12 Kohlenstoffatomen, wobei die genannten Kohlenwasserstoffreste unsubstituiert oder mit 1 bis 3 Methoxy-, Thiomethoxy- oder Dimethylamino-Gruppen substituiert sein können, und die beiden Reste R¹ und R² unter Bildung eines, das Phosphoratom einschließenden 5 bis 10-Ringes auch miteinander verbunden sein können.

Im Fall eines aliphatischen Kohlenwasserstoffrests kann dieser unverzweigt oder verzweigt beziehungsweise linear oder cyclisch sein. Bevorzugt besitzen die aliphatischen Kohlenwasserstoffrest 1 bis 6 Kohlenstoffatome, besonders bevorzugt 1 bis 4 Kohlenstoffatome und ganz besonders bevorzugt 1 bis 2 Kohlenstoffatome. Als konkrete Beispiele seien genannt Methyl, Ethyl, iso-Propyl, n-Propyl, n-Butyl, iso-Butyl, tert.-Butyl (auch als tBu bezeichnet) und Cyclohexyl (auch als Cy bezeichnet).

Im Fall eines aromatischen Kohlenwasserstoffrests handelt es sich um Phenyl (auch als Ph bezeichnet), 1-Naphthyl oder 2-Naphthyl.

Araliphatischen Kohlenwasserstoffreste enthalten aromatische und aliphatische Elemente, unabhängig davon, ob diese über eine aliphatische oder eine aromatische Gruppe an das Phosphoratom im Liganden L gebunden sind. Bevorzugt besitzen die araliphatischen Kohlenwasserstoffreste 7 bis 10 Kohlenstoffatome und besonders bevorzugt 7 bis 9 Kohlenstoffatome. Als konkrete Beispiele seien genannt o-Tolyl, m-Tolyl, p-Tolyl und Benzyl.

Im Fall eines, das Phosphoratom einschließenden Ringes, handelt es sich bevorzugt um einen Ring mit 5 bis 6 Atomen, einschließlich des Phosphoratoms. Als Beispiele seien genannt Butan-1,4-diyl, Pentan-1,5-diyl und 2,4-Dimethylpentan-1,5-diyl.

Die genannten aliphatischen, aromatischen und araliphatischen Kohlenwasserstoffreste, die auch unter Bildung eines, das Phosphoratom einschließenden Ringes miteinander verbunden sein können, können unsubstituiert oder mit 1 bis 3 Methoxy-, Thiomethoxy- oder Dimethylamino-Gruppen substituiert sein. Die oben genannte Anzahl der Kohlenstoffatome der einzelnen Kohlenwasserstoffreste ist einschließlich der Kohlenstoffatome der Methoxy-, Thiomethoxybeziehungsweise Dimethylamino-Gruppen zu verstehen. Als konkrete Beispiele seien genannt 3,5-Dimethyl-phenyl, 3,5-Dimethyl-4-methoxy-phenyl, 3,5-Dimethyl-4-thiomethoxy-phenyl und 3,5-Dimethyl-4-(dimethylamino)phenyl.

Besonders bevorzugt handelt es sich bei den Resten R¹ und R² Phenyl, p-Tolyl, o-Tolyl, 4-Methoxyphenyl, 2-Methoxyphenyl, Cyclohexyl, iso-Butyl, tert-Butyl, 3,5-Dimethyl-4-methoxyphenyl, 3,5-tert-Butyl-4-methoxyphenyl und 3,5-Dimethyl-phenyl und ganz besonders bevorzugt um Phenyl, p-Tolyl, 3,5-Dimethyl-4-methoxyphenyl, iso-Butyl und Cyclohexyl, wobei bevorzugt jeweils beide Reste gleich sind.

Die Reste R³, R⁴, R⁵, R⁶, R¹⁰ und R¹¹ stehen unabhängig voneinander für Wasserstoff, lineares C₁ bis C₄-Alkyl, verzweigtes C₃ bis C₄-Alkyl, Methoxy, Hydroxy, Trifluormethyl, Nitril oder Dialkylamino mit unabhängig voneinander 1 bis 4 Kohlenstoffatomen je Alkylgruppe. Als lineares C₁ bis C₄-Alkyl sind Methyl, Ethyl, n-Propyl und n-Butyl sowie als verzweigtes C₃ bis C₄-Alkyl iso-Propyl, sec.-Butyl und tert.-Butyl zu nennen. Als Dialkylamino sind insbesondere Amino-Reste mit identischen Alkylgruppen zu nennen, insbesondere Dimethylamino, Diethylamino, Di-n-propylamino und Di-n-butylamino.

Bevorzugt stehen die Reste R³ und R⁴ unabhängig voneinander für Wasserstoff oder Methyl und besonders bevorzugt für Wasserstoff.

Bevorzugt steht der Rest R⁵ für Wasserstoff, Methyl, iso-Propyl, sec.-Butyl, tert.-Butyl, Methoxy, Hydroxy oder Dialkylamino, besonders bevorzugt für Wasserstoff, Methyl oder Hydroxy und ganz besonders bevorzugt für Wasserstoff.

Bevorzugt steht der Rest R⁶ für Wasserstoff.

Bevorzugt steht der Rest R¹⁰ für Wasserstoff, Methyl, iso-Propyl, sec.-Butyl, tert.-Butyl oder Methoxy, besonders bevorzugt für Wasserstoff, Methyl oder tert.-Butyl und ganz besonders bevorzugt für Wasserstoff.

Bevorzugt steht der Rest R¹¹ für Wasserstoff, Methyl, Ethyl, Methoxy, Ethoxy oder iso-Propyloxy und besonders bevorzugt für Wasserstoff, Methyl oder Methoxy.

Ganz besonders bevorzugt sind Liganden (II), bei denen
- R³, R⁴, R⁵, R⁶, R¹⁰ und R¹¹ für Wasserstoff stehen,
- R³, R⁴, R⁵, R⁶ und R¹⁰ für Wasserstoff und R¹¹ für Methyl stehen,
- R³, R⁴, R⁵, R⁶ und R¹⁰ für Wasserstoff und R¹¹ für Methoxy stehen,
- R³, R⁴, R⁶, R¹⁰ und R¹¹ für Wasserstoff und R⁵ für Methyl stehen,
- R³, R⁴, R⁶, R¹⁰ und R¹¹ für Wasserstoff und R⁵ für tert.-Butyl stehen
- R³, R⁴, R⁵, R⁶ und R¹¹ für Wasserstoff und R¹⁰ für Methyl stehen,
- R³, R⁴, R⁵, R⁶ und R¹¹ für Wasserstoff und R¹⁰ für tert.-Butyl stehen, sowie
- R³, R⁴, R⁵ und R⁶ für Wasserstoff und R¹⁰ und R¹¹ für Methyl stehen.

Die Reste R⁷, R⁸ und R⁹ stehen unabhängig voneinander für Wasserstoff, lineares C₁ bis C₄-Alkyl oder verzweigtes C₃ bis C₄-Alkyl. Als lineares C₁ bis C₄-Alkyl sind Methyl, Ethyl, n-Propyl und n-Butyl sowie als verzweigtes C₃ bis C₄-Alkyl iso-Propyl, sec.-Butyl und tert.-Butyl zu nennen.

Bevorzugt stehen die Reste R⁷, R⁸ und R⁹ unabhängig voneinander für Wasserstoff, Methyl, Ethyl oder n-Propyl, besonders bevorzugt für Wasserstoff oder Methyl und ganz besonders bevorzugt für Wasserstoff.

Ganz besonders bevorzugt sind Liganden (II), bei denen
- R⁷, R⁸ und R⁹ für Wasserstoff stehen,
- R⁷ und R⁹ für Wasserstoff und R⁸ für Methyl stehen,
- R⁷ für Wasserstoff und R⁸ und R⁹ für Methyl stehen,
- R⁷ für Methyl und R⁸ und R⁹ für Wasserstoff stehen, und
- R⁷ und R⁸ für Methyl und R⁹ für Wasserstoff stehen.

Besonders vorteilhaft beim erfindungsmäßen Verfahren ist der Einsatz der Liganden (II), bei denen
(i) n und m jeweils 1 ist und die beiden durchgezogen-gestrichelten Doppellinien eine Ein fachbindung darstellen (Struktur (IIa)), oder
(ii) n gleich 0 und m gleich 1 ist und die dem Phenylring zugewandte durchgezogen-gestrichelte Doppellinie eine Doppelbindung und die dem Pyridylring zugewandte durch gezogen-gestrichelte Doppellinie eine Einfachbindung darstellt (Struktur (IIb)),
   und

- beide Reste R¹ und R² für Phenyl, p-Tolyl, 3,5-Dimethyl-4-methoxyphenyl, iso-Butyl oder Cyclohexyl stehen,
- die Reste R³, R⁴ und R⁶ für Wasserstoff stehen,
- die Reste R⁵ und R¹⁰ für Wasserstoff, Methyl oder tert.-Butyl stehen,
- der Rest R¹¹ für Wasserstoff, Methyl oder Methoxy steht, und
- die Reste R⁷, R⁸ und R⁹ für Wasserstoff oder Methyl stehen.

Als besonders geeignet sind demgemäß beim erfindungsgemäßen Verfahren die Liganden L1, L2, L3, L4 und L5.

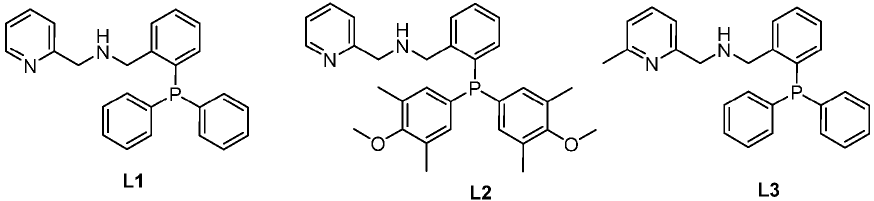

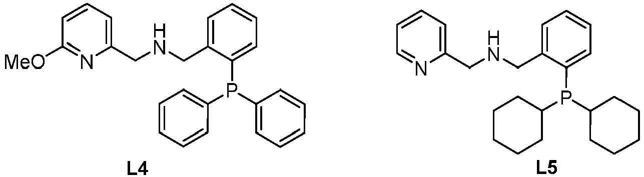

Ligand (II) kann auf einfache Art und Weise durch Kondensation eines entsprechenden Amins mit einem entsprechenden Aldehyd oder Keton (Ligand (IIb) und (Ilc)) sowie einer eventuell nachfolgenden Reduktion (Ligand (IIa)) sowie einer eventuell nachfolgenden Deprotonierung unter basischen Bedingungen (Ligand (Ild)) erhalten werden.

Bei der Kondensation gibt es grundsätzlich zwei verschiedene Möglichkeiten. Zum einen ist es möglich, als Aminkomponente 2-Picolylamin oder ein entsprechendes Derivat davon, und als Aldehyd- oder Ketonkomponente einen entsprechend substituierten Phosphanylbenzaldehyd oder ein entsprechendes Keton einzusetzen.

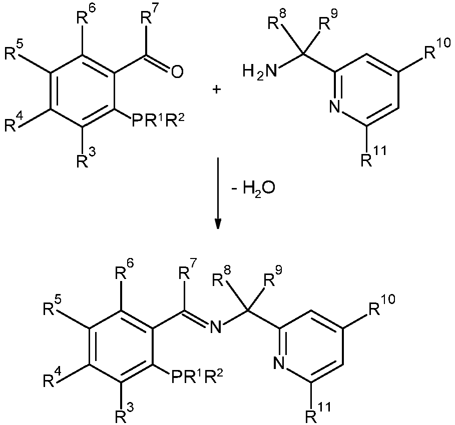

### Ligand (Ilb)

Zum anderen ist es aber auch möglich, als Aminkomponente ein entsprechend substituiertes Phosphanylphenylmethanamin und als Aldehyd- oder Ketonkomponente Picolinaldehyd oder ein entsprechendes Derivat davon einzusetzen.

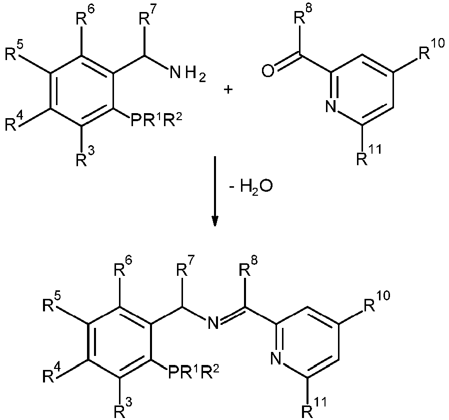

### Ligand (IIc)

Die entsprechenden Ausgangsverbindungen (Amine, Ketone oder Aldehyde) sind in der Regel kommerziell erhältlich oder können mit allgemein bekannten Methoden synthetisiert werden. Die Synthese der Liganden (IIb) und (IIc) erfolgt üblicherweise unter Schutzgasatmosphäre. Die **beiden Komponenten** werden dabei üblicherweise in einem Lösungsmittel bei einer Temperatur von 50 bis 200°C miteinander umgesetzt. Als geeignete Lösungsmittel seien beispielhaft aliphatische Alkohole wie beispielsweise Methanol, Ethanol oder Isopropanol sowie aromatische Kohlenwasserstoffe wie etwa Toluol oder Xylole genannt. Die beiden Ausgangsverbindungen können in stöchiometrischer Menge eingesetzt werden. Es ist aber auch möglich, eine der beiden Komponenten im Überschuss einzusetzen, um beispielsweise den Umsatz der anderen Komponente zu erhöhen. Dies ist insbesondere sinnvoll, wenn die andere Komponente schwer zugänglich ist. Wird ein Überschuss eingesetzt, so liegt das Molverhältnis der beiden Ausgangsverbindungen in der Regel im Bereich von > 1 bis ≤ 2. Als Reaktionszeit reichen üblicherweise wenige Minuten bis mehrere Stunden. Als typische Reaktionszeit seien 10 Minuten bis 5 Stunden und bevorzugt 30 Minuten bis 3 Stunden genannt. Die Aufarbeitung des Reaktionsgemisches und Isolierung des Liganden können nach üblichen Methoden erfolgen. Vorteilhafterweise entfernt man jedoch das zugesetzte Lösungsmittel und Wasser im Vakuum.

Die Liganden (IIb) und (IIc) können nun zur Darstellung des Mangan(I)-Komplexes (I) eingesetzt werden.

Durch Reduktion des Liganden (IIb) oder (IIc) mit Reduktionsmitteln wie beispielsweise Natriumborhydrid oder Lithiumaluminiumhydrid oder katalytisch mit Wasserstoff kann aus den Liganden (IIb) und (IIc) in einfacher Art und Weise der Ligand (IIa) gewonnen werden. Die Umsetzung kann mit dem üblichen Wissen des Fachmanns durchgeführt werden.

In einer besonders vorteilhaften Synthese führt man die oben beschriebene Kondensation und die Reduktion zum Liganden (IIa) in einer Eintopfreaktion direkt hintereinander durch, ohne die Liganden (IIb) und (IIc) vorab zwischen zu isolieren. Hierzu gibt man nach dem Ablauf der Kondensation das Reduktionsmittel direkt zum Reaktionsgemisch und lässt es für eine weitere Zeit miteinander reagieren. Auch hierzu reichen üblicherweise wenige Minuten bis mehrere Stunden. Als typische Reaktionszeit seien 10 Minuten bis 5 Stunden und bevorzugt 30 Minuten bis 3 Stunden genannt. Anschließend kann die Aufarbeitung des Reaktionsgemisches und Isolierung des Liganden nach üblichen Methoden erfolgen. Auf die zur Aufarbeitung und Isolierung der Liganden (IIb) und (IIc) genannten Angaben sei explizit hingewiesen.

Ligand (IIa) kann sich aus den Liganden (IIb) und (IIc) auch gebunden im Mangan(I)-Komplex (I) unter Reaktionsbedingungen durch Hydrierung mit dem zugeführten Wasserstoff bilden.

Der anionische Ligand (IId) bildet sich aus dem Liganden (IIa) durch Umsetzung mit einer starken Base infolge einer Abspaltung des am Stickstoff befindlichen Wasserstoffatoms als Proton.

Geeignete starke Basen sind beispielsweise NaOMe, NaOEt, KOEt, KOt-Bu oder KOMe. Üblicherweise führt man diese Umsetzung nicht gezielt mit dem freien Liganden (IIa) durch. Vielmehr kann sich der Ligand (Ild) im Mangan(I)Komplex (I) unter Hydrierbedingungen in Gegenwart einer starken Base bilden.

Der beim erfindungsgemäßen Verfahren einzusetzende Mangan(I)-Komplex (I) trägt neben dem Ligand II noch mindestens zwei Carbonylliganden (=CO).

Beim erfindungsgemäßen Verfahren ist die Oxidationsstufe des Mangans +1.

Der beim erfindungsgemäßen Verfahren bevorzugt eingesetzte Mangan(I)-Komplex (I) besitzt die allgemeine Formel (I)

[Mn(L)(CO)₂₊ₙX₁₋ₙ]Z₍ₙ₎ (I)

wobei
X für einen anionischen monodentaten Liganden mit der Ladung "-1" steht
Z für ein anionisches Gegenion mit der Ladung "-1" steht
n für 0 oder 1 steht
aufweist.

Der Index n gibt an, ob der Mangan(I)Komplex (I) zwei (n = 0) oder drei CO-Liganden (n = 1) trägt. Ist n = 0, so befindet so ist der anionische Ligand am Mangan. Ist n = 1, so ist der Mangankomplex kationisch und die Ladung wir durch das anionische Gegenion Z ausgeglichen.

Als Mangan(I)-Komplex (I) ist beim erfindungsgemäßen Verfahren ein Ruthenium-Komplex bevorzugt, bei dem
- X: für einen anionischen Liganden ausgewählt aus der Gruppe H⁻, F⁻, Cl⁻, Br⁻, I⁻, OH⁻, C₁ bis C₆-Alkoxy, C₁ bis C₆-Carboxy, Methylallyl, Acetylacetonato, RSO₃⁻, CF₃SO₃', CN⁻ und BH₄⁻, bevorzugt Br⁻ oder Cl⁻ steht
- Z: für einen anionisches Gegenion ausgewählt aus der Gruppe F⁻, Cl⁻, Br⁻, I⁻, OH⁻, C₁ bis C₆-Alkoxy, C₁ bis C₆-Carboxy, Methylallyl, Acetylacetonato, RSO₃, CF₃SO₃⁻, CN⁻, BH₄⁻, BF₄⁻, PF₆⁻ CIO₄⁻, NO₃⁻, BPh₄⁻, bevorzugt Br⁻, CI⁻, C₁ bis C₆-Alkoxy und C₁ bis C₆-Carboxy steht.

Als bevorzugte Beispiele zu Mangan(I)-Komplexen (I) seien genannt [Mn(L)(CO)₂Br], [Mn(L)(CO)₂CI], [Mn(L)(CO)₂I], [Mn(L)(CO)₂OMe], [Mn(L)(CO)₂CN], [Mn(L)(CO)₂OH], [Mn(L)(CO)₂H], [Mn(L)(CO)₃][Br], [Mn(L)(CO)₃][Cl], [Mn(L)(CO)₃][I], [Mn(L)(CO)₃][OtBu], [Mn(L)(CO)₃][CN], [Mn(L)(CO)₃][NO₃], [Mn(L)(CO)₃][CIO₄], [Mn(L)(CO)₃][BF₄], [Mn(L)(CO)₃][PF₆] wobei L jeweils für die neutralen Liganden (IIa), (IIb) oder (IIc) steht.

Ganz besonders bevorzugt führt man das erfindungsgemäße Verfahren in Gegenwart von Mangan(I)Komplexen (I) durch, bei denen
- der Ligand (II) für den Liganden (IIa), (IIb) oder (IIc) steht und darin
- die Reste R¹, R² jeweils für Phenyl, p-Tolyl, 3,5-Dimethyl-4-methoxyphenyl, iso-Butyl oder Cyclohexyl stehen,
- die Reste R⁵ und R¹⁰ unabhängig voneinander für Wasserstoff, Methyl oder tert.-Butyl stehen,
- der Rest R¹¹ unabhängig voneinander für Wasserstoff, Methyl oder Methoxy steht,
- die Reste R⁷, R⁸ und R⁹ unabhängig voneinander für Wasserstoff oder Methyl stehen.

Als besonders geeignet sind demgemäß beim erfindungsgemäßen Verfahren die Mangan(I)-Komplexe (I) K1, K2, K3, K4 und K5.

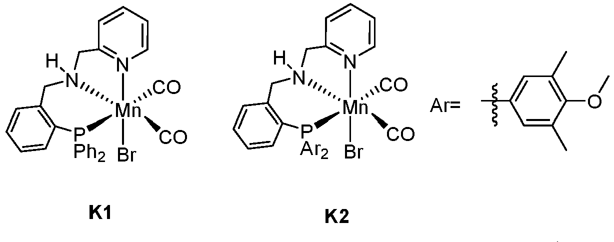

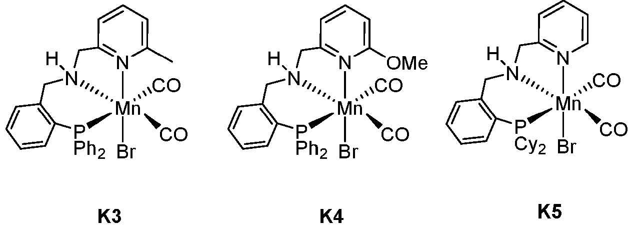

Die beim erfindungsgemäßen Verfahren einzusetzenden Mangan(I)-Komplexe (I) können auf verschiedene Art und Weise erhalten werden. Eine bevorzugte Möglichkeit besteht darin, als Mangan-haltigen Ausgangsstoff eine Verbindung einzusetzen, in der das Mangan bereits in Form eines Komplexes vorliegt, im Folgenden als Mn-Precursorkomplex (IV) bezeichnet, und diesen mit dem Liganden L umsetzt. Entsprechend ist ein Verfahren bevorzugt, bei dem man den Mangan(I)-Komplex (I) durch Umsetzung von Ligand (II) mit einem Mn-Precursorkomplex (IV) erhält.

Als Mn-Precursorkomplex (IV) können grundsätzlich die verschiedensten Mn-Komplexe eingesetzt werden. Dabei ist es in vielen Fällen nicht erforderlich, dass der Mn-Precursorkomplex (IV) bereits die Liganden X oder Carbonyl sowie gegebenenfalls das nicht-koordinierende Anion Z des gewünschten Mangan-Komplexe (I) enthält. Der Liganden X, CO sowie das nicht-koordinierende Anion Z können in vielen Fällen dem Syntheseansatz auch separat zugeführt werden. Um den Syntheseaufwand niedrig zu halten, setzt man vorteilhafterweise als Mn-Precursorkomplexe (IV) leicht zugängliche beziehungsweise leicht verfügbare Komplexe ein. Dem Fachmann sind derartige Komplexe wohlbekannt. Ebenso ist der Fachmann auch mit dem Austausch von Liganden an Mangan-haltigen Komplexen vertraut.

Als anionische Liganden im Mn-Precursorkomplex (IV) kommen grundsätzlich alle anionischen Liganden in Frage, welche bereits unter dem Liganden X beschrieben sind.

Die Umsetzung des Mn-Precursorkomplexes (IV) mit dem Liganden L führt man üblicherweise bei einem Mn/L-Molverhältnis von 0,8 bis 20 bevorzugt von 0,9 bis 10 und besonders bevorzugt von 0,9 bis 1,1 durch. Im Sinne eines möglichst hohen Umsetzungsgrades ist es dabei vorteilhaft, einen Mn-Precursorkomplex (IV) mit nur ein- und zweizähnigen Liganden einzusetzen, um den Komplexierungseffekt des dreizähnigen Liganden L zu nutzen. Üblicherweise führt man die Umsetzung wasserfrei, jedoch in Gegenwart eines Lösungsmittels und unter einer Schutzgasatmosphäre durch. Als geeignete Lösungsmittel seien beispielhaft aliphatische Alkohole wie beispielsweise Methanol, Ethanol oder Isopropanol sowie aromatische Kohlenwasserstoffe wie etwa Toluol oder Xylole genannt. Im Allgemeinen weist Mangan im Mn-Precursorkomplex (IV) dieselbe Oxidationsstufe auf wie im anschließenden Mangan(I)-Komplex (I), also die Oxidationsstufe +I.

Als geeignete Mn-Precursorkomplexe (IV) seien beispielsweise [Mn(CO)₅Br], [Mn(CO)₅CI], [Mn(CO)₅I], [Mn(CO)₅F], [Mn₂(CO)₁₀], MnCl₂, Mn(OAc)₂, Mn(OAc)₃, Mn(AcAc)₂, Mn(AcAc)₃, MnBr₂, MnI₂, MnCO₃, Mn(NO₃)₂ und Mn(CIO₄)₂ genannt. (AcAc=Acetylacetonat)

Der Mangan-Komplex (I) kann aus dem erhaltenen Reaktionsgemisch beispielsweise durch Ausfällen oder Kristallisation isoliert werden.

Zur Durchführung der erfindungsgemäßen Hydrierung ist es jedoch im Allgemeinen nicht erforderlich, den Mangan(I)-Komplex (I) nach dessen Herstellung erst zu isolieren. Vielmehr ist es im Sinne einer vereinfachten Verfahrensführung vorteilhaft, den Mangan(I)-Komplex (I) wie oben beschrieben aus einem Mn-Precursorkomplex (IV) und dem Liganden L in Gegenwart eines Lösungsmittels herzustellen und die erfindungsgemäße Hydrierung direkt in dem erhaltenen Reaktionsgemisch durchzuführen.

Im erfindungsgemäßen Verfahren können auch verschiede Mangan(I)-Komplexe (I), besonders auch Gemische von kationischen- und neutralen Mangan(I)-Komplex (I) die den gleichen Liganden L (II) enthalten.

Die beim erfindungsgemäßen Verfahren einzusetzenden Ester können vielfältiger Natur sein. So können grundsätzlich lineare oder verzweigte, nichtcyclische oder cyclische, gesättigte oder ungesättigte, aliphatische, aromatische oder araliphatische, unsubstituierte oder durch Heteroatome oder funktionelle Gruppen unterbrochene Ester unterschiedlicher Molmassen von niedermolekular bis hochmolekular eingesetzt werden.

Bevorzugt setzt man als Ester einen Ester der allgemeinen Formel (III) 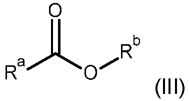 ein, bei dem die Reste R^{a} und R^{b} jeweils unabhängig voneinander für einen Kohlenstoff enthaltenden organischen, linearen oder verzweigten, nichtcyclischen oder cyclischen, gesättigten oder ungesättigten, aliphatischen, aromatischen oder araliphatischen, unsubstituierten oder durch Heteroatome oder funktionelle Gruppen unterbrochenen oder substituierten Rest mit einer Molmasse von 15 bis 10000 g/mol stehen, wobei beide Reste R^{a} und R^{b} auch miteinander verbunden sein können.

Im Falle von verzweigten Resten R^{a} und R^{b} können diese einfach oder mehrfach verzweigt sein. Ebenso können im Falle von cyclischen Resten, diese einfach oder mehrfach cyclisch sein. Desgleichen können im Falle von ungesättigten Resten diese einfach oder mehrfach ungesättigt sein, wobei hierbei sowohl Doppelbindungen als auch Dreifachbindungen möglich sind. Als Heteroatome sind Atome zu verstehen, welche weder Kohlenstoff noch Wasserstoff sind. Als bevorzugte Beispiele von Heteroatomen seien genannt Sauerstoff, Stickstoff, Schwefel, Phosphor, Fluor, Chlor, Brom und lod, sowie als besonders bevorzugte Beispiele Sauerstoff, Stickstoff, Fluor, Chlor und Brom. Funktionelle Gruppen sind eine weitere Umschreibung von Gruppen, welche mindestens ein Heteroatom enthalten. So kann beispielsweise eine mittels -O- unterbrochene Kohlenwasserstoffkette sowohl als eine Kohlenwasserstoffkette betrachtet werden, die durch ein Sauerstoff-Heteroatom unterbrochen ist, als auch als eine Kohlenwasserstoffkette, die durch eine Ether-Gruppe unterbrochen ist. Als weitere, nicht einschränkende Beispiele seien etwa Amino-Gruppen (-NH₂, -NH-, -N<), Aldehyd-Gruppen (-CHO), Carboxy-Gruppen (-COOH), Amid-Gruppen (-CONH₂, -CONH-, -CON<), Nitril-Gruppen (-CN), Isonitril-Gruppen (-NC), Nitro-Gruppen (-NO₂), Sulfonsäure-Gruppen (-SO₃), Keto-Gruppen (>CO), IminoGruppen (>CNH, >CN-), Ester-Gruppen (-CO-O-), Anhydrid-Gruppen (-CO-O-CO-) und Imido-Gruppen (-CO-NH-CO-, -CO-NR-CO-) genannt. Es können natürlich auch mehrere sogenannter funktioneller Gruppen enthalten sein. Als Beispiel hierfür seien etwa Fette genannt.

Sind die Reste R^{a} und R^{b} miteinander verbunden, liegen cyclische Ester vor, die auch als Lactone bezeichnet werden.

Die Molmassen der Reste R^{a} und R^{b} betragen im Allgemeinen 15 bis 10000 g/mol, bevorzugt 15 bis 5000 g/mol und besonders bevorzugt 15 bis 2000 g/mol.

Bevorzugt setzt man beim erfindungsgemäßen Verfahren Ester mit einer Molmasse von 74 bis 20000 g/mol, besonders bevorzugt von 74 bis 10000 g/mol, ganz besonders bevorzugt von 74 bis 5000 g/mol, vor allem 74 bis 2000 g/mol und insbesondere von 74 bis 1000 g/mol ein.

Der beim erfindungsgemäßen Verfahren einzusetzende molekulare Wasserstoff (H₂) kann sowohl unverdünnt als auch mit Inertgas, beispielsweise Stickstoff, verdünnt zugeführt werden. Vorteilhaft ist die Zufuhr eines Wasserstoff-enthaltenden Gases mit einem möglichst hohen Gehalt an Wasserstoff. Bevorzugt ist ein Gehalt an Wasserstoff von ≥ 80 Vol.-%, besonders bevorzugt von ≥ 90 Vol.-%, ganz besonders bevorzugt von ≥ 95 Vol.-% und insbesondere von ≥ 99 Vol.-%.

Beim erfindungsgemäßen Verfahren führt man in einer ganz allgemeinen Ausführungsform einem geeigneten Reaktionsapparat den Mangan(I)-Komplex (I), den zu hydrierenden Ester sowie Wasserstoff zu und setzt das Gemisch unter den gewünschten Reaktionsbedingungen um.

Als Reaktionsapparate können beim erfindungsgemäßen Verfahren grundsätzlich Reaktionsapparate eingesetzt werden, die für gas/flüssig-Reaktionen unter der gegebenen Temperatur und dem gegebenen Druck grundsätzlich geeignet sind. Geeignete Standardreaktoren für gas/flüssig- und für flüssig/flüssig-Reaktionssyteme sind beispielsweise in K.D. Henkel, "Reactor Types and Their Industrial Applications", in Ullmann's Encyclopedia of Industrial Chemistry, 2005, Wiley-VCH Verlag GmbH & Co. KGaA, DOI: 10.1002/14356007.b04_087, Kapitel 3.3 "Reactors for gas-liquid reactions" beschrieben. Als Beispiele seien genannt Rührkesselreaktoren, Rohrreaktoren oder Blasensäulenreaktoren. Druckresistente Rührkessel werden in der Regel auch als Autoklaven bezeichnet.

Der Mangan(I)-Komplex (I) kann dabei direkt in Form des vorab synthetisierten Mangan(I)-Komplexes (I) dem Reaktionsapparat zugeführt werden.

Deutlich einfacher ist es, wenn man den Mangan(I)-Komplex (I) in-situ aus einem Mn-Precursorkomplex (IV) und Ligand L (II) bildet. In-situ steht für "vor Ort" und bedeutet, dass man den Mangan(I)-Komplex (I) durch Zufuhr von Mn-Precursorkomplex (IV) und Ligand L (II) im Reaktionsapparat bildet. Vorteilhafterweise setzt man hierzu ein Molverhältnis von Ligand L (II) zu Mangan von 0,5 bis 5, bevorzugt ≥ 0,8 und besonders bevorzugt ≥ 1, sowie bevorzugt ≤ 3, besonders bevorzugt ≤ 2 und ganz besonders bevorzugt ≤ 1,5 ein. Diese in-situ Variante erspart das vorherige Isolieren des Mangan(I)-Komplexes (I).

Das erfindungsgemäße Verfahren kann in Gegenwart aber auch in Abwesenheit eines Lösungsmittels erfolgen. Wird ein Lösungsmittel eingesetzt, so dient dieses beispielsweise zur Lösung des Mangan(I)-Komplexes (I) beziehungsweise eines Mn-Precursorkomplexes (IV) sowie des Liganden L, aber auch gegebenenfalls zur Lösung des zu hydrierenden Esters. Vor allem bei niedermolekularen Estern kann auch dieser als Lösungsmittel fungieren.

Werden Lösungsmittel eingesetzt, so kommen bevorzugt Lösungsmittel mit mehr oder minder stark ausgeprägten polaren Eigenschaften in Frage, die unter den Reaktionsbedingungen nicht selbst hydriert werden. Als bevorzugte Beispiele seien genannt aliphatische Alkohole wie beispielsweise Methanol, Ethanol oder Isopropanol sowie aromatische Kohlenwasserstoffe wie etwa Toluol oder Xylole und Ether wie Tetrahydrofuran oder 1,4-Dioxan. Die Menge an eingesetztem Lösungsmittel kann breit variieren. Üblich sind jedoch Mengen im Bereich von 0,1 bis 20 g Lösungsmittel pro g zu hydrierendem Ester, bevorzugt 0,5 bis 10 g Lösungsmittel pro g zu hydrierendem Ester und besonders bevorzugt 1 bis 5 g Lösungsmittel pro g zu hydrierendem Ester.

Der zu hydrierende Ester kann direkt in Form des reinen, unverdünnten Esters, aber auch verdünnt beziehungsweise in einem Lösungsmittel gelöst zugeführt werden. Kriterien, in welcher Form der zu hydrierende Ester zugegeben wird, sind in der Regel oftmals rein praktischer Natur, wie beispielsweise die Natur des vorliegenden Esters sowie dessen Handhabung. So ist es beispielsweise anzustreben, dass das der Ester im Reaktionsgemisch unter den Reaktionsbedingungen in flüssiger Form vorliegt.

Das Molverhältnis zwischen dem zu hydrierenden Ester und dem Mangan-Komplex (I) kann beim erfindungsgemäßen Verfahren in einem breiten Bereich variieren. Im Allgemeinen beträgt das genannte Molverhältnis in dem zu hydrierenden Reaktionsgemisch 1 bis 100000, bevorzugt 10 bis 25000, besonders bevorzugt 100 bis 5000 und ganz besonders bevorzugt 500 bis 20000.

Das erfindungsgemäße Verfahren führt man bei einer Temperatur von 50 bis 200°C, bevorzugt bei ≤ 170°C und besonders bevorzugt bei ≤ 150°C durch. Der Druck beträgt dabei 0,1 bis 20 MPa abs, bevorzugt ≥ 1 MPa abs und besonders bevorzugt ≥ 5 MPa abs, sowie bevorzugt ≤ 15 MPa abs und besonders bevorzugt ≤ 10 MPa abs.

Die Reaktionszeit beziehungsweise mittlere Verweilzeit, in der das Reaktionsgemisch unter den Reaktionsbedingungen vorliegt, kann ebenfalls breit variieren, liegt jedoch üblicherweise im Bereich von 0,1 bis 100 Stunden, bevorzugt bei ≥ 1 Stunden und besonders bevorzugt bei ≥ 2 Stunden, sowie bevorzugt bei ≤ 80 Stunden und besonders bevorzugt bei ≤ 60 Stunden.

Des Weiteren hat sich gezeigt, dass in der Regel die erfindungsgemäße Hydrierung durch Gegenwart einer Base positiv beeinflusst wird und dadurch letztendlich deutlich höhere Umsätze ermöglicht werden. Daher ist es in den meisten Fällen vorteilhaft, die Hydrierung in Gegenwart einer Base durchzuführen. Grundsätzlich können die Basen im Reaktionsgemisch auch als Feststoff vorliegen, bevorzugt sind jedoch Basen, die im Reaktionsgemisch gelöst vorliegen. Als Beispiele möglicher Basen seien genannt Alkoholate, Hydroxide, Alkalimetall- und Erdalkalimetall-Carbonate, Amide, basische Aluminium und Silicium Verbindungen sowie Hydride. Besonders bevorzugt setzt man als Base Alkoholate oder Amide, bevorzugt Natriummethanolat, Kaliummethanolat, Natriumhydroxid, Natriumethylat, Kaliumethylat, KaliumtertButylat, Natriumtert-Butylat, Natriumborhydrid oder Natriumhydrid ein.

Wird das erfindungsgemäße Verfahren in Gegenwart einer Base durchgeführt, so wird diese in Bezug auf den Mangan(I)Komplex (I) im Allgemeinen im Überschuss eingesetzt. Bevorzugt setzt man ein Molverhältnis der Base zum Ruthenium-Komplex (I) von 1 bis 1000, bevorzugt von 2 bis 20, besonders bevorzugt von 1 bis 10 ein.

Das erfindungsgemäße Verfahren kann kontinuierlich, in semibatch-Fahrweise, diskontinuierlich, rückvermischt in Produkt als Lösungsmittel oder im geraden Durchgang nicht rückvermischt durchgeführt werden. Die Zuleitung des Mangan(I)-Komplexes, des zu hydrierenden Esters, des Wasserstoffs, gegebenenfalls des Lösungsmittels und gegebenenfalls der Base kann dabei simultan oder getrennt voneinander erfolgen.

Bei der diskontinuierlichen Fahrweise legt man üblicherweise den Mangan-Komplex (I) beziehungsweise einen Mn-Precursorkomplex (IV) sowie den Liganden L (II), den zu hydrierenden Ester und gegebenenfalls Lösungsmittel und eine Base im Reaktionsapparat vor und stellt unter den gewünschten Reaktionsbedingungen unter Durchmischung durch Zugabe von Wasserstoff den gewünschten Reaktionsdruck ein. Anschließend belässt man das Reaktionsgemisch während der gewünschten Reaktionszeit unter den gewünschten Reaktionsbedingungen. Gegebenenfalls dosiert man Wasserstoff nach. Nach Ablauf der gewünschten Reaktionszeit kühlt man das Reaktionsgemisch ab beziehungsweise entspannt es. Durch eine nachfolgende Aufarbeitung können die korrespondierenden Alkohole als Reaktionsprodukt erhalten werden. Bevorzugt führt man die diskontinuierliche Umsetzung in einem Rührkessel durch.

Bei der kontinuierlichen Fahrweise führt man dem Reaktionsapparat den Mangan(I)-Komplex (I) beziehungsweise einen Mn-Precursorkomplex (IV) sowie den Liganden L (II), den zu hydrierenden Ester und gegebenenfalls Lösungsmittel und eine Base kontinuierlich zu und entnimmt kontinuierlich eine entsprechende Menge zur Aufarbeitung und Isolierung des gebildeten korrespondierenden Alkohols.

Bevorzugt führt man die kontinuierliche Umsetzung in einem Rührkessel oder einer Rührkesselkaskade durch.

Das Hydrierprodukt lässt sich durch den Fachmann an sich bekannte Verfahren, wie zum Beispiel durch Destillation und/oder Entspannungsverdampfung, aus dem Hydriergemisch abtrennen und der zurückbleibende Katalysator im Rahmen weiterer Umsetzungen nutzen. Im Rahmen der bevorzugten Ausführungsform verzichtet man vorteilhafterweise auf den Zusatz von Lösungsmitteln und führt die genannten Umsetzungen im umzusetzenden Substrat beziehungsweise dem Produkt und gegebenenfalls in hochsiedenden Nebenprodukten als Lösemedium durch. Insbesondere bevorzugt ist die kontinuierliche Reaktionsführung unter Wiederverwendung beziehungsweise Rückführung des homogenen Katalysators.

Bei der erfindungsmäßen Esterhydrierung werden aus der -CO-O- Estergruppe eine endständige -CH₂OH und eine endständige -OH Gruppe gebildet. Im Falle des Esters (III) bilden sich somit entsprechend der nachfolgenden Reaktionsgleichung die beiden korrespondierenden Alkohole R^{a}-CH₂OH und R^{b}-OH. 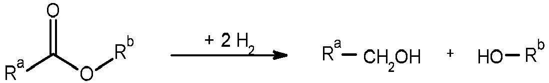 Ester (III)

Beim Einsatz cyclischer Ester, sogenannter Lactone, sind die beiden Reste R^{a} und R^{b} miteinander verbunden und es bildet sich das entsprechende Diol.

Das erfindungsgemäße Verfahren ermöglicht die Herstellung von Alkoholen durch homogenkatalysierte Hydrierung von Estern in hoher Ausbeute und Selektivität. Die Hydrierung ist technisch in üblichem Laborgerät für Hydrierreaktionen durchführbar und ermöglicht den Einsatz der unterschiedlichsten Ester als Substrate.

Eine weitere Ausführungsform der Erfindung ist die Umsetzung von Sclareolid zu Ambroxidol (Ambrox-1,4-diol), welches eine Vorstufe zu dem wichtigen Riechstoff (-) Ambrox darstellt. Ambroxidol kann durch Cyclisierung, wie sie z.B. in WO 2017/140909 beschrieben ist, zu (-) Ambrox umgesetzt werden.

Die besonderen Vorteile des erfindungsgemäßen Verfahrens basieren auf den speziellen, dreizähnigen PNN-Liganden. Durch seine Dreizähnigkeit koordiniert der Ligand fest am Mangan, ist aber einfach herzustellen und liefert nach der Bildung entsprechende Mangan(I)-Komplexe Katalysatoren mit hohen Hydrieraktivitäten. Zudem ist der erfindungsgemäße Ligand auch relativ oxidationsunempfindlich, so dass dieser auch in der Handhabung vorteilhaft ist und eine hohe Lagerstabilität aufweist.

Unter den besonderen Vorteilen des erfindungsgemäßen Liganden ist vor allem dessen leichte Zugänglichkeit und leichte Variationsmöglichkeit der Grundstruktur durch Ersatz von Wasserstoffatomen durch diverse organische Reste zu nennen. Der Ligand kann in der Regel aus gut verfügbaren Einsatzstoffen durch eine einfache Eintopfsynthese hergestellt werden. Als Mangan-haltige Einsatzstoffe können leicht zugängliche und kommerziell in großen Mengen verfügbare Mangan-Precursorkomplexe eingesetzt werden.

### Beispiele

### Generelle Informationen

Wenn nicht anders angegeben, wurden alle Reaktionen unter einer Argon-Atmosphäre unter Verwendung sogenannter "Schlenk"- und Hochvakuum-Techniken oder in einer MBraun Inert Atmosphären Handschuhbox bei Raumtemperatur vorbereitet. Organische Lösemittel wurden von Aldrich oder Acros bezogen. Kommerziell erhältliche Ausgangsverbindungen wurden von Aldrich, ABCR oder TCI bezogen und eingesetzt wie erhalten. NMR Spektren wurden auf Bruker AVANCE III 300, Bruker AVANCE III 400 und Bruker AVANCE III 500 Spektrometer gemessen und als Referenz dienten die Protonen (¹H) oder Kohlenstoff (¹³C) Resonanzsignale des Lösemittels. Chemische Verschiebungen (δ) sind in ppm angegeben. ³¹P NMR Spektren beziehen sich auf einen externen Standard (Ampulle D₃PO₄) des Organisch-Chemischen Institutes der Universität Heidelberg. GC Analysen wurden auf einem Agilent Technologies 6890N Gaschromatograph ausgestattet mit einem FID Detektor durchgeführt; Verwendete Säule: DB-FFAP (30 m×0.32 mm×0.25 µm. Initialtemperatur - 55 °C, Haltezeit-1 min; Rampe 25 °C/min bis 250 °C, Haltezeit-8 min.

In Beispiel 1 ist die Herstellung eines repräsentativen Liganden II beschrieben. Die anderen Liganden wurden analog dieser Vorschrift hergestellt.

### Beispiel 1: Herstellung von Ligand L1

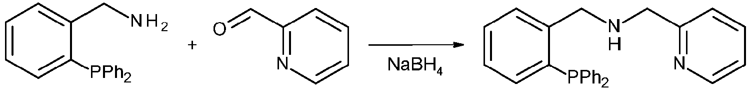

Bei Raumtemperatur wurde zu einer Lösung von Picolinaldehyd (368 mg, 3.43 mmol) in Ethanol (10 ml) (2-(Diphenylphosphaneyl)phenyl)methanamin (1.00 g, 3.43 mmol) gegeben und das resultierende Gemisch 2 h bei Raumtemperatur gerührt. NaBH₄ (208 mg, 5,49 mmol) wurde zugegeben und weitere 2 h bei Raumtemperatur gerührt. Anschließend wurde wässrige gesättigte NaHCO₃-Lösung (15 ml) und CH₂Cl₂ (25 ml) zugegeben. Nach Phasentrennung wurde die wässrige Phase mit CH₂CI₂ (2 x 25 ml) extrahiert. Die vereinigte organische Phase wurde getrocknet (Na₂SO₄) und im Vakuum eingeengt. Das Rohprodukt wurde säulenchromatographisch an Kieselgel gereinigt (Hexan/EtOAc/NEt₃, 9:1 bis 1:1; es wurde eine Mischung von 10% NEt₃ in EtOAc verwendet) und N-(2-(Diphenylphosphaneyl)benzyl)-1-(pyridin-2-yl)methanamin (L1) als farbloses Öl (600 mg, 46% Ausbeute) erhalten.

¹H **NMR** (500 MHz, CD₂CI₂) δ 8.48-8.46 (m, 1H), 7.57 (td, J= 7.7, 1.8 Hz, 1H), 7.54-7.51 (m, 1H), 7.36-7.30 (m, 7H), 7.28-7.24 (m, 4H), 7.19-7.10 (m, 4H), 6.91 (ddd, J= 7.7, 4.5, 1.4 Hz, 1H), 4.02 (d, *J =* 1.7 Hz, 2H), 3.79 (s, 2H).

³¹P **NMR** (203 MHz, CD₂CI₂) δ -15.94.

**HRMS (ESI)** C₂₅H₂₃N₂P ([M]⁺): Berechnet: 382.1599; Gefunden: 382.1611.

In Beispiel 2 ist die Herstellung eines repräsentativen Mangan(I)-Katalysatorkomplex (I) mit einem Liganden II beschrieben.

### Beispiel 2: Herstellung von Katalysator K1

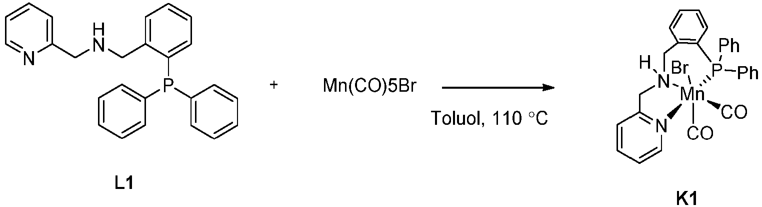

Zu einer Lösung des Liganden L1 (1 g, 2.62 mmol) in 15 ml Toluol werden unter einer Argon Atmosphäre eine Lösung von [Mn(CO)₅Br] (720 mg, 2.62 mmol) in 15 ml Toluol zugegeben. Das Reaktionsgemisch wird bei 60-80°C für 30 Minuten gerührt, bis sich keine sichtbare CO-Entwicklung mehr zu beobachten ist. Dann wird das Gemisch weitere 16 Stunden bei 110°C gerührt, wobei ein gelber Niederschlag entsteht. Nach dem Abkühlen auf Raumtemperatur wird das Toluol im Vakuum vom Reaktionsgemisch abgezogen. Der gelbe Rückstand wird mit 10 ml absolutem Hexan und dann mit 10 ml absolutem Diethylether unter einer Argonatmosphäre gewaschen und abfiltriert. Der Filterkuchen wird im Hochvakuum getrocknet und man erhält die Katalysator K1 als gelbes Pulver mit einer Ausbeute von 80% (1.2 g). Der Katalysatorkomplex wird unter Lichtabschluss bei 0°C gelagert.

¹H **NMR** (500 MHz, CD₂CI₂) δ 9.09 (d, *J* = 5.5 Hz, 1H), 8.07 (s, 2H), 7.67 (t, *J =* 7.1 Hz, 1H), 7.57 - 7.22 (m, 11H), 7.05 (t, *J* = 8.5 Hz, 2H), 6.81 (t, *J* = 8.2 Hz, 1H), 4.59 - 3.59 (m, 5H).

¹³C **NMR** (126 MHz, CD₂CI₂) δ 154.62, 151.77, 135.76 (d, *J =* 16.5 Hz), 133.55 - 133.09 (m), 129.76, 129.56, 129.00, 128.79 (d, *J =* 3.2 Hz), 128.70, 127.93 (d, *J =* 8.4 Hz), 127.18 (dd, *J* = 7.9, 2.2 Hz), 126.13 (d, *J =* 5.2 Hz), 125.92 (d, *J =* 2.0 Hz), 124.93 (d, *J =* 9.0 Hz), 124.63 (d, *J* = 9.5 Hz), 120.93, 117.01, 56.86 (d, *J =* 2.6 Hz), 55.71 (d, *J =* 8.1 Hz).

³¹P **NMR** (203 MHz, CD₂CI₂) δ 68.39.

### Beispiel 3: Herstellung von Katalysator K2

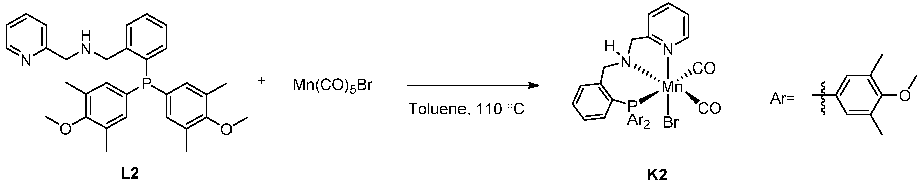

Zu einer Lösung des Liganden L2 (0.155 g, 0.31 mmol) in 10 ml Toluol werden unter einer Argon Atmosphäre eine Lösung von [Mn(CO)₅Br] (85 mg, 0.31 mmol) in 10 ml Toluol zugegeben. Das Reaktionsgemisch wird bei 60-80°C für 30 Minuten gerührt, bis sich keine sichtbare CO-Entwicklung mehr zu beobachten ist. Dann wird das Gemisch weitere 16 Stunden bei 110°C gerührt, wobei ein gelber Niederschlag entsteht. Nach dem Abkühlen auf Raumtemperatur wird das Toluol im Vakuum vom Reaktionsgemisch abgezogen. Der gelbe Rückstand wird mit 10 ml absolutem Hexan und dann mit 10 ml absolutem Diethylether unter einer Argonatmosphäre gewaschen und abfiltriert. Der Filterkuchen wird im Hochvakuum getrocknet und man erhält die Katalysator K2 als gelbes Pulver mit einer Ausbeute von 75% (0.16 g). Der Katalysatorkomplex wird unter Lichtabschluss bei 0°C gelagert.

### Beispiel 4: Herstellung von Katalysator K3

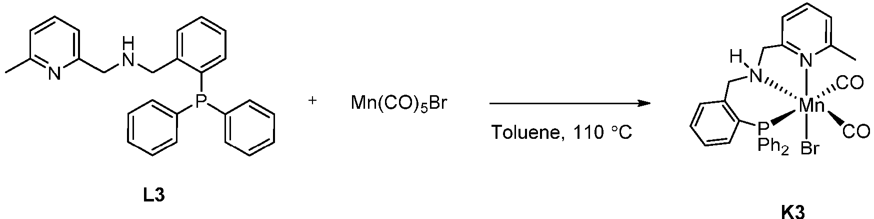

Zu einer Lösung des Liganden L3 (0.14 g, 0.35 mmol) in 10 ml Toluol werden unter einer Argon Atmosphäre eine Lösung von [Mn(CO)₅Br] (97 mg, 0.35 mmol) in 10 ml Toluol zugegeben. Das Reaktionsgemisch wird bei 60-80°C für 30 Minuten gerührt, bis sich keine sichtbare CO-Entwicklung mehr zu beobachten ist. Dann wird das Gemisch weitere 16 Stunden bei 110°C gerührt, wobei ein gelber Niederschlag entsteht. Nach dem Abkühlen auf Raumtemperatur wird das Toluol im Vakuum vom Reaktionsgemisch abgezogen. Der gelbe Rückstand wird mit 10 ml absolutem Hexan und dann mit 10 ml absolutem Diethylether unter einer Argonatmosphäre gewaschen und abfiltriert. Der Filterkuchen wird im Hochvakuum getrocknet und man erhält die Katalysator K2 als gelbes Pulver mit einer Ausbeute von 56% (0.115 g). Der Katalysatorkomplex wird unter Lichtabschluss bei 0°C gelagert.

### Beispiel 5: Herstellung von Katalysator K4

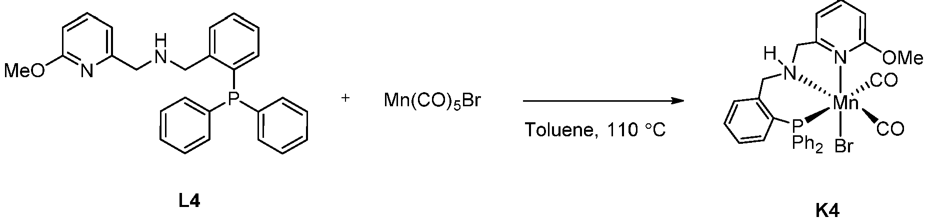

Zu einer Lösung des Liganden L4 (0.54 g, 1.31 mmol) in 10 ml Toluol werden unter einer Argon Atmosphäre eine Lösung von [Mn(CO)₅Br] (0.36 g, 1.31 mmol) in 10 ml Toluol zugegeben. Das Reaktionsgemisch wird bei 60-80°C für 30 Minuten gerührt, bis sich keine sichtbare CO-Entwicklung mehr zu beobachten ist. Dann wird das Gemisch weitere 16 Stunden bei 110°C gerührt, wobei ein gelber Niederschlag entsteht. Nach dem Abkühlen auf Raumtemperatur wird das Toluol im Vakuum vom Reaktionsgemisch abgezogen. Der gelbe Rückstand wird mit 10 ml absolutem Hexan und dann mit 10 ml absolutem Diethylether unter einer Argonatmosphäre gewaschen und abfiltriert. Der Filterkuchen wird im Hochvakuum getrocknet und man erhält die Katalysator K2 als gelbes Pulver mit einer Ausbeute von 82% (0.65 g). Der Katalysatorkomplex wird unter Lichtabschluss bei 0°C gelagert.

### Beispiel 5: Herstellung von Katalysator K5

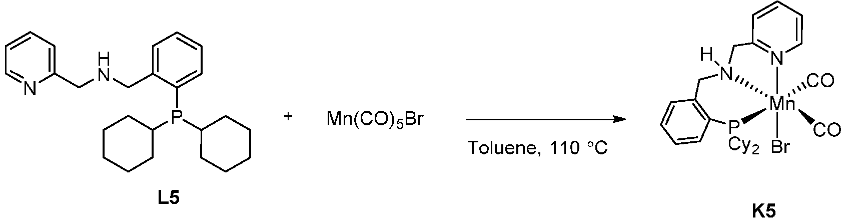

Zu einer Lösung des Liganden L5 (0.34 g, 0.86 mmol) in 10 ml Toluol werden unter einer Argon Atmosphäre eine Lösung von [Mn(CO)₅Br] (0.236 g, 0.86 mmol) in 10 ml Toluol zugegeben. Das Reaktionsgemisch wird bei 60-80°C für 30 Minuten gerührt, bis sich keine sichtbare CO-Entwicklung mehr zu beobachten ist. Dann wird das Gemisch weitere 16 Stunden bei 110°C gerührt, wobei ein gelber Niederschlag entsteht. Nach dem Abkühlen auf Raumtemperatur wird das Toluol im Vakuum vom Reaktionsgemisch abgezogen. Der gelbe Rückstand wird mit 10 ml absolutem Hexan und dann mit 10 ml absolutem Diethylether unter einer Argonatmosphäre gewaschen und abfiltriert. Der Filterkuchen wird im Hochvakuum getrocknet und man erhält die Katalysator K2 als gelbes Pulver mit einer Ausbeute von 75% (0.38 g). Der Katalysatorkomplex wird unter Lichtabschluss bei 0°C gelagert.

### Beispiele 6, 7, 8 und 9: Hydrierung Sclareolid

In einer Argon-gefüllten Handschuhbox werden in 10mL Gläschen mit Bördelkappen und PTFE-beschichteten Magnetrührstäbchen Sclareolid (375 mg, 1.5 mmol), Mangankatalysator (0.1 mol%), KOtBu (3.36 mg, 2 mol%) und trockenes Ethanol (2 ml) eingefüllt. Die Gläschen werden mit der Bördelkappe, die ein Gummiseptum enthält verschlossen, das Septum mit einer Kanüle durchstochen und die Gläschen werden in einem HEL CAT-7 Autoklav platziert. Der Autoklav wird verschlossen, aus der Handschuhbox entnommen und unter Inertbedingungen werden 50 bar H₂ aufgepresst und der Autoklav in einen vorgeheizten Aluminiumblock eingeführt. Das Reaktionsgemisch wird bei 100°C für 20 h gerührt, in einem Eisbad abgekühlt und der verbliebene H₂-Druck wird vorsichtig abgelassen. Dann wird zu den jeweiligen Ansätzen Mesitylen als interner Standard zugegeben und die Reaktionsgemische werden per Gaschromatographie analysiert.

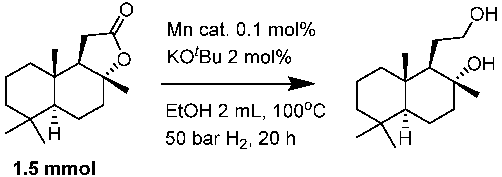

| **Beispiel** | Katalysator | Umsatz | **GC Ausbeute Diol** |
|---|---|---|---|
| 6 | **K1** | full | 93% |
| 7 | **K2** | full | 91.5% |
| 8 | **K4** | 34% | 34% |
| 9 | **K5** | full | 94% |

### Beispiel 10: Hydrierung von Sclareolid

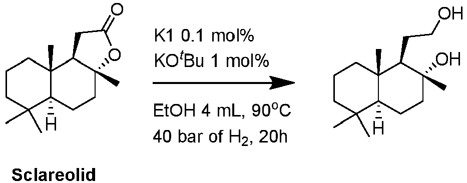

In einer Argon-gefüllten Handschuhbox werden unter Inertbedingungen Sclareolid (750 mg, 3 mmol), K1 (1.7 mg, 0.003 mmol), KaliumtertButylat (3.36 mg, 0.003 mmol) und 4 ml trocknes Ethanol in einen 100 ml Premex-Autoklaven mit Teflon-Einsatz und Magnetrührstab eingewogen. Der Autoklav wird verschlossen und ausgeschleust. Dann wird dieser dreimal mit Stickstoff und dann dreimal mit Wasserstoff gespült und mit Wasserstoff auf 40 bar Kaltdruck aufgepresst. Dann wird das Reaktionsgemisch im Autoklav für 20 h bei 90°C gerührt Rühren erhitzt. Nach dem Abkühlen auf Raumtemperatur wird der verbliebene Wasserstoff abgelassen und das Reaktionsgemisch mittels GC analysiert. Umsatz Sclareolid: 98%, Ausbeute des Diols: 92%.
**(2R,8aS)-1-(2-hydroxyethyl)-2,5,5,8a-tetramethyldecahydronaphthalen-2-ol**
**¹H NMR** (400 MHz, CDCl;) δ 3.78 (dt, *J =* 10.2, 4.4 Hz, 1H), 3.46 (ddd, *J =* 10.2, 8.2, 5.7 Hz, 1H), 3.08 (s, 2H), 1.90 (dt, *J =* 12.3, 3.3 Hz, 1H), 1.72 - 1.21 (m, 10H), 1.19 (s, 3H), 1.13 (dd, *J* = 13.4, 4.4 Hz, 1H), 0.99 - 0.91 (m, 2H), 0.88 (s, 3H), 0.79 (s, 6H).
**¹³C NMR** (101 MHz, CDCl:) δ 73.03, 64.09, 59.18, 56.04, 44.29, 41.91, 39.36, 38.98, 33.41, 33.28, 27.89, 24.64, 21.48, 20.47, 18.42, 15.31.

### Beispiel 11: Hydrierung von Sclareolid

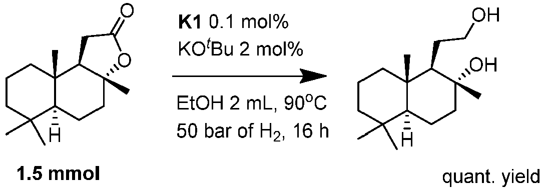

In einer Argon-gefüllten Handschuhbox werden in ein 10 ml Gläschen mit Bördelkappen und PTFE-beschichteten Magnetrührstäbchen Sclareolid (375 mg, 1.5 mmol), Mangankatalysator K1 (0.1 mol%), KOtBu (3.36 mg, 2 mol%) und trockenes Ethanol (2 ml) eingefüllt. Die Gläschen werden mit der Bördelkappe, die ein Gummiseptum enthält verschlossen, das Septum mit einer Kanüle durchstochen und die Gläschen werden in einem HEL CAT-7 Autoklav platziert. Der Autoklav wird verschlossen, aus der Handschuhbox entnommen und unter Inertbedingungen werden 50 bar H₂ aufgepresst und der Autoklav in einen vorgeheizten Aluminiumblock eingeführt. Das Reaktionsgemisch wird bei 90°C für 16 h gerührt, in einem Eisbad abgekühlt und der verbliebene H₂-Druck wird vorsichtig abgelassen. Das Reaktionsgemisch wird über Silica filtriert und das Silica mehrfach mit Ethanol gewaschen. Das Ethanol wird von den vereinigten Filtraten im Vakuum bis zur Trockene abgezogen und das Produkt per NMR analysiert. Die isolierte Ausbeute an Sclareoldidiol ist quantitativ (Ausbeute >99%) und gemäß 1H NMR Spektrum handelt es sich um Reinsubstanz.

### Beispiel 12: Hydrierung von Sclareolid

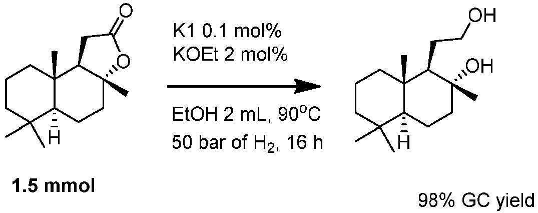

In einer Argon-gefüllten Handschuhbox werden unter Inertbedingungen Sclareolid (375 mg, 1.5 mmol), K1 (0.88 mg, 0.1 mol%), Kaliumethylat (2.52 mg, 2 mol%) und 2 ml trocknes Ethanol in einen 30 ml Premex-Autoklaven mit Teflon-Einsatz und Magnetrührstab eingewogen. Der Autoklav wird verschlossen und ausgeschleust. Dann wird dieser dreimal mit Stickstoff und dann dreimal mit Wasserstoff gespült und mit Wasserstoff auf 40 bar Kaltdruck aufgepresst. Dann wird das Reaktionsgemisch im Autoklav für 16 h bei 90°C gerührt Rühren erhitzt. Nach dem Abkühlen auf Raumtemperatur wird der verbliebene Wasserstoff abgelassen und das Reaktionsgemisch mittels GC analysiert. Umsatz Sclareolid: >99%, Ausbeute des Diols: 98%.

### Beispiele 11, 12, 13, 14 und 15: Hydrierung anderer Ester mit dem Katalysator K1

In einer Argon-gefüllten Handschuhbox werden in 10mL Gläschen mit Bördelkappen und PTFE-beschichteten Magnetrührstäbchen der jeweilige Ester (3 mmol), Mangankatalysator K1 (1.72 mg, 0.1 mol%), KOtBu (6.72 mg, 2 mol%) und trockenes Ethanol (4 ml) eingefüllt. Die Gläschen werden mit der Bördelkappe, die ein Gummiseptum enthält verschlossen, das Septum mit einer Kanüle durchstochen und die Gläschen werden in einem HEL CAT-7 Autoklav platziert. Der Autoklav wird verschlossen, aus der Handschuhbox entnommen und unter Inertbedingungen werden 50 bar H₂ aufgepresst und der Autoklav in einen vorgeheizten Aluminiumblock eingeführt. Das Reaktionsgemisch wird bei 100°C für 20 h gerührt, in einem Eisbad abgekühlt und der verbliebene H₂-Druck wird vorsichtig abgelassen. Die Reaktionsmischungen werden über Silica filtriert und das Silica mehrfach mit Ethanol gewaschen. Das Ethanol wird von den vereinigten Filtraten im Vakuum bis zur Trockene abgezogen und das Produkt per NMR analysiert.

| | |
|---|---|
| 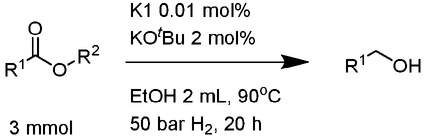 | 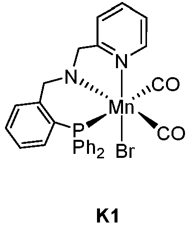 |

| | |
|---|---|
| 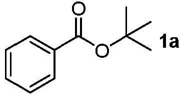 Beispiel 11 | 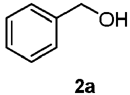 Ausbeute 99% |
| 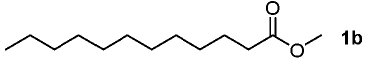 Beispiel 12 | 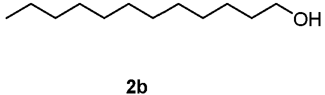 Ausbeute 99% |
| 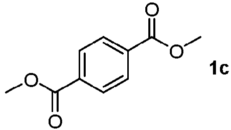 Beispiel 13 | 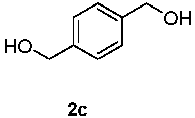 Ausbeute 99% |
| 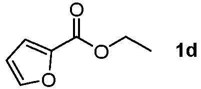 Beispiel 14 | 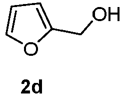 Ausbeute 96% |
| 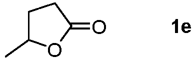 Beispiel 15 | 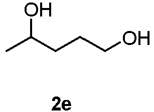 Ausbeute 99% |

### Phenylmethanol, 2a

**¹H NMR** (301 MHz, CDCl₃) δ 7.39 - 7.26 (m, 5H), 4.66 (s, 2H), 1.98 (s, 1H). **¹³C NMR** (76 MHz, CDCl₃) δ 140.89, 128.58, 127.66, 127.02, 65.34.

### Dodecan-1-ol, 2b

**¹H NMR** (301 MHz, CDCl₃) δ 3.64 (t, *J =* 6.6 Hz, 2H), 1.55 (q, *J =* 7.1 Hz, 2H), 1.37-1.26 (m, 18H), 0.93 - 0.83 (m, 3H).

**¹³C NMR** (76 MHz, CDCl₃) δ 63.09, 32.82, 31.92, 29.67, 29.64, 29.62, 29.61, 29.45, 29.35, 25.75, 22.69, 14.11.

### 1,4-phenylenedimethanol, 2c

**¹H NMR** (301 MHz, CDCl₃) δ 7.37 (s, 4H), 4.70 (s, 4H), 1.64 (s, 2H). **¹³C NMR** (76 MHz, CDCl₃) δ 127.25, 88.96.

### Furan-2-ylmethanol, 2d

**¹H NMR** (301 MHz, CDCl₃) δ 7.39 (s, 1H), 6.31 (d, *J* = 15.0 Hz, 2H), 4.58 (s, 2H), 2.27 (s, 1H). **¹³C NMR** (76 MHz, CDCl₃) δ 154.03, 142.57, 110.36, 107.76, 57.42.

### Pentane-1,4-diol, 2e

**¹H NMR** (301 MHz, CDCl₃) δ 3.94 - 3.78 (m, 1H), 3.77 - 3.58 (m, 2H), 2.72 (s, 2H), 1.76 - 1.39 (m, 4H), 1.21 (d, *J* = 6.2 Hz, 3H).

**¹³C NMR** (76 MHz, CDCl₃) δ 67.95, 62.89, 36.26, 29.14, 23.60.

## Patentansprüche

1. Verfahren zur Hydrierung eines Esters der allgemeinen Formel (III) 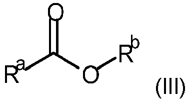
bei dem die Reste R^{a} und R^{b} jeweils unabhängig voneinander für einen Kohlenstoff enthaltenden organischen, linearen oder verzweigten, nichtcyclischen oder cyclischen, gesättigten oder ungesättigten, aliphatischen, aromatischen oder arylaliphatischen, unsubstituierten oder durch Heteroatome oder funktionelle Gruppen unterbrochenen oder substituierten Rest mit einer Molmasse von 15 bis 10000 g/mol stehen, wobei beide Reste R^{a} und R^{b} auch miteinander verbunden sein können,
mit molekularem Wasserstoff zu den Alkoholen 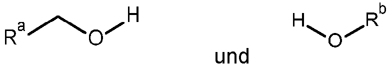
bei einer Temperatur von 50 bis 200°C und einem Druck von 0,1 bis 20 MPa abs in Gegenwart eines Mangan(I)-Komplexes, bei dem der Mangan-Komplex einen dreizähnigen Liganden L mit der allgemeinen Formel (II) 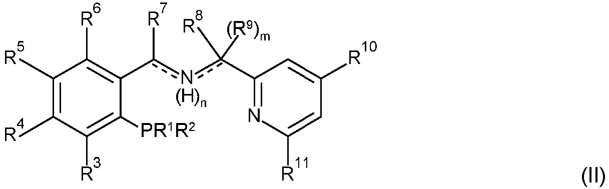
sowie mindestens zwei Carbonylliganden enthält, wobei
R¹, R² unabhängig voneinander für einen aliphatischen Kohlenwasserstoffrest mit 1 bis 8 Kohlenstoffatomen, einen aromatischen Kohlenwasserstoffrest mit 6 oder 10 Kohlenstoffatomen oder einen araliphatischen Kohlenwasserstoffrest mit 7 bis 12 Kohlenstoffatomen stehen, wobei die genannten Kohlenwasserstoffreste unsubstituiert oder mit 1 bis 3 Methoxy-, Thiomethoxy- oder Dimethylamino-Gruppen substituiert sind, und die beiden Reste R¹ und R² unter Bildung eines, das Phosphoratom einschließenden 5 bis 10-Ringes auch miteinander verbunden sein können,
R³, R⁴, R⁵, R⁶, R¹⁰, R¹¹ unabhängig voneinander für Wasserstoff, lineares C₁ bis C₄-Alkyl, verzweigtes C₃ bis C₄-Alkyl, Methoxy, Hydroxy, Trifluormethyl, Nitril oder Dialkylamino mit unabhängig voneinander 1 bis 4 Kohlenstoffatomen je Alkylgruppe, stehen,
R⁷, R⁸, R⁹ unabhängig voneinander für Wasserstoff, lineares C₁ bis C₄-Alkyl oder verzweigtes C₃ bis C₄-Alkyl stehen, n, m unabhängig voneinander 0 oder 1 sind, und die durchgezogen-gestrichelten Doppellinien für eine Einfach- oder Doppelbindung stehen, mit der Maßgabe, dass
| | |
|---|---|
| im Falle von n = 1 | beide durchgezogen-gestrichelten Doppellinien eine Einfachbindung darstellen und m gleich 1 ist, und |
| im Falle von n = 0 | eine durchgezogen-gestrichelte Doppellinie eine Einfachbindung und die andere durchgezogen-gestrichelte Doppellinie eine Doppelbindung darstellt, wobei im Falle einer Doppelbindung auf der dem Phenylring zu gewandten Seite m = 1 ist, im Falle einer Doppelbindung auf der dem Pyridylring zugewandten Seite m = 0 ist, oder beide durchgezogen-gestrichelten Doppellinien eine Einfachbindung darstellen und m gleich 1 ist. |

2. Verfahren nach Anspruch 1, **dadurch gekennzeichnet, dass** der Mangan-Komplex die allgemeine Formel (I)
[Mn(L)(CO)₂₊ₙX₁₋ₙ] Z₍ₙ₎ (I)
wobei
X für einen anionischen monodentaten Liganden mit der Ladung "-1" steht
Z für ein anionisches Gegenion mit der Ladung "-1" steht
n für 0 oder 1 steht
aufweist.

3. Verfahren nach den Ansprüchen 1 und 2 **dadurch gekennzeichnet, dass** man ein Molverhältnis zwischen dem Ester und dem Mangan(I)-Komplex I von 100 bis 100 000 einsetzt.

4. Verfahren nach den Ansprüchen 1 bis 3, **dadurch gekennzeichnet, dass** man die Reaktion in Gegenwart einer Base als Cokatalysator durchführt.

5. Verfahren nach den Ansprüchen 1 bis 4, **dadurch gekennzeichnet, dass** der eingesetzte Ester III Sclareolid ist, welches zum entsprechenden Diol hydriert wird.

6. Verfahren zur Herstellung von (-) Ambrox, **dadurch gekennzeichnet, dass** Sclareolid in einem ersten Schritt (i) gemäß Anspruch 5 zu Ambrox-1,4-diol hydriert wird, und das erhaltene Ambrox-1,4-diol in einem zweiten Schritt (ii) zu (-) Ambrox zyklisiert wird.

## Claims

1. A method for hydrogenating an ester of the general formula (III) 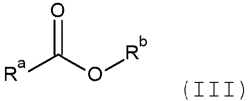
in which the radicals R^{a} and R^{b} are each independently a carbon-containing organic, linear or branched, noncyclic or cyclic, saturated or unsaturated, aliphatic, aromatic or araliphatic radical which is unsubstituted or interrupted or substituted by heteroatoms or functional groups and has a molar mass of 15 to 10 000 g/mol, wherein the two radicals R^{a} and R^{b} may also be bonded to each other,
with molecular hydrogen to give the alcohols 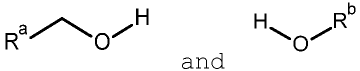
at a temperature of 50 to 200°C and a pressure of 0.1 to 20 MPa abs in the presence of a manganese(I) complex, in which the manganese complex comprises a tridentate ligand L of the general formula (II) 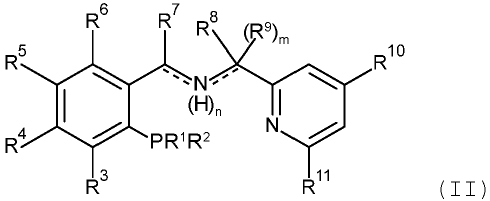
and comprises at least two carbonyl ligands, wherein
R¹, R² are each independently an aliphatic hydrocarbon radical having 1 to 8 carbon atoms, an aromatic hydrocarbon radical having 6 or 10 carbon atoms or an araliphatic hydrocarbon radical having 7 to 12 carbon atoms, where the hydrocarbon radicals specified are unsubstituted or substituted by 1 to 3 methoxy, thiomethoxy or dimethylamino groups, and the two radicals R¹ and R² may also be bonded to each other to form a 5-to 10-membered ring including the phosphorus atom,
R³, R⁴, R⁵, R⁶, R¹⁰, R¹¹ are each independently hydrogen, linear C₁ to C₄-alkyl, branched C₃ to C₄-alkyl, methoxy, hydroxyl, trifluoromethyl, nitrile or dialkylamino each independently having 1 to 4 carbon atoms per alkyl group, R⁷, R⁸, R⁹ are each independently hydrogen, linear C₁ to C₄-alkyl or branched C₃ to C₄-alkyl, n, m are each independently 0 or 1, and the solid-dashed double lines are a single or double bond, with the proviso that
in the case of n = 1, both solid-dashed double lines are a single bond and m is 1, and
in the case of n = 0, one solid-dashed double line is a single bond and the other solid-dashed double line is a double bond, wherein, in the case of a double bond on the side facing the phenyl ring m = 1, in the case of a double bond on the side facing the pyridyl ring m = 0, or both solid-dashed double lines are a single bond and m equals 1.

2. The method according to claim 1, wherein the manganese complex has the general formula (I)
[Mn(L)(CO)₂₊ₙX₁₋ₙ] Z₍ₙ₎ (I)
wherein
X is an anionic monodentate ligand having a charge of "-1"
Z is an anionic counterion having a charge of "-1"
n is 0 or 1.

3. The method according to claims 1 and 2, wherein a molar ratio between the ester and the manganese (I) complex I of 100 to 100 000 is used.

4. The method according to claims 1 to 3, wherein the reaction is carried out in the presence of a base as cocatalyst.

5. The method according to claims 1 to 4, wherein the ester III used is sclareolide, which is hydrogenated to the corresponding diol.

6. A method for producing (-) Ambrox, wherein sclareolide is hydrogenated to Ambrox-1,4-diol in a first step (i) according to claim 5, and the resulting Ambrox-1,4-diol is cyclized to (-) Ambrox in a second step (ii).

## Revendications

1. Procédé d'hydrogénation d'un ester de formule générale (III) 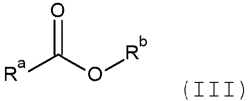
dans laquelle les radicaux R^{a} et R^{b} représentent, à chaque fois indépendamment l'un de l'autre, un radical organique contenant du carbone, linéaire ou ramifié, non cyclique ou cyclique, saturé ou insaturé, aliphatique, aromatique ou arylaliphatique, non substitué ou interrompu ou substitué par des hétéroatomes ou des groupes fonctionnels, présentant une masse molaire de 15 à 10.000 g/mole, les deux radicaux R^{a} et R^{b} pouvant également être reliés l'un à l'autre.
avec de l'hydrogène moléculaire en alcools 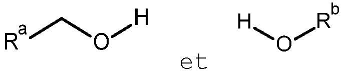
à une température de 50 à 200°C et à une pression de 0,1 à 20 MPa abs en présence d'un complexe de manganèse (I), dans lequel le complexe de manganèse contient un ligand tridentate L de la formule générale (II) 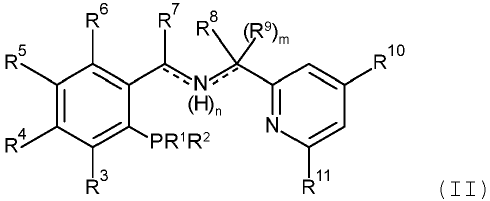
ainsi qu'au moins deux ligands carbonyle,
R¹, R² représentant, indépendamment l'un de l'autre, un radical hydrocarboné aliphatique comprenant 1 à 8 atomes de carbone, un radical hydrocarboné aromatique comprenant 6 ou 10 atomes de carbone ou un radical hydrocarboné araliphatique comprenant 7 à 12 atomes de carbone, les radicaux hydrocarbonés mentionnés étant non substitués ou substitués par 1 à 3 groupes méthoxy, thiométhoxy ou diméthylamino et les deux radicaux R¹ et R² pouvant également être reliés l'un à l'autre avec formation d'un cycle de 5 à 10 chaînons, incluant l'atome de phosphore, R³, R⁴, R⁵, R⁶, R¹⁰, R¹¹ représentant, indépendamment les uns des autres, hydrogène, C₁-C₄-alkyle linéaire, C₃-C₄-alkyle ramifié, méthoxy, hydroxy, trifluorométhyle, nitrile ou dialkylamino comprenant, indépendamment, 1 à 4 atomes de carbone par groupe alkyle,
R⁷, R⁸, R⁹ représentant, indépendamment les uns des autres, hydrogène, C₁-C₄-alkyle linéaire ou C₃-C₄-alkyle ramifié, n, m représentant, indépendamment l'un de l'autre, 0 ou 1 et les doubles lignes continues-pointillées représentant une simple ou une double liaison, étant entendu que
dans le cas de n = 1, les deux doubles lignes continues-pointillées représentent une simple liaison et m vaut 1 et
dans le cas de n = 0, une double ligne continue-pointillée représente une simple liaison et l'autre double ligne continue-pointillée représente une double liaison, où, dans le cas d'une double liaison sur le côté orienté vers le cycle phényle, m = 1, dans le cas d'une double liaison orientée vers le cycle pyridyle, m = 0, ou les deux doubles lignes continues-pointillées représentent une simple liaison et m vaut 1.

2. Procédé selon la revendication 1, **caractérisé en ce que** le complexe de manganèse présente la formule générale (I)
[Mn(L)(CO)₂₊ₙX₁₋ₙ] Z₍ₙ₎ (I)
dans laquelle
X représente un ligand monodentate anionique de charge "-1",
Z représente un contre-ion anionique de charge "-1",
n représente 0 ou 1.

3. Procédé selon les revendications 1 et 2, **caractérisé en ce qu'**on utilise un rapport molaire entre l'ester et le complexe de manganèse (I) I de 100:100.000.

4. Procédé selon les revendications 1 à 3, **caractérisé en ce qu'**on effectue la réaction en présence d'une base en tant que cocatalyseur.

5. Procédé selon les revendications 1 à 4, **caractérisé en ce que** l'ester III utilisé est le sclaréolide qui est hydrogéné en diol correspondant.

6. Procédé de préparation de (-)ambrox, **caractérisé en ce que** le sclaréolide est hydrogéné dans une première étape (i) selon la revendication 5 en ambrox-1,4-diol et l'ambrox-1,4-diol obtenu est cyclisé dans une deuxième étape (ii) en (-)ambrox.
